# EUROPEAN PATENT APPLICATION

(11) **EP 4 786 467 A1**
(43) Date of publication of application: **05.08.2026**
(21) Application number: 24871021.2
(22) Date of filing: 27.09.2024
(51) Int. Cl.: C07D 407/14, C07D 409/12, A61K 31/4709, A61P 25/00

(54) **QUINOLINONE COMPOUND, PREPARATION METHOD THEREFOR AND USE THEREOF**

(30) Priority: 28.09.2023 CN 202311277525
(71) Applicant: Vigonvita Life Sciences Co., Ltd., Suzhou, Jiangsu 215123 (CN)
(72) Inventor: SHEN, Jingshan, Suzhou, Jiangsu 215123 (CN); HE, Yang, Suzhou, Jiangsu 215123 (CN); WU, Chunhui, Suzhou, Jiangsu 215123 (CN); JI, Jing, Suzhou, Jiangsu 215123 (CN); HU, Tianwen, Suzhou, Jiangsu 215123 (CN); TIAN, Guanghui, Suzhou, Jiangsu 215123 (CN)
(74) Representative: Kador & Partner Part mbB
(86) International application number: PCT/CN2024/121920
(87) International publication number: WO 2025/067486

(57) **Abstract**

Disclosed in the present invention are a quinolinone compound, a preparation method therefor and the use thereof. Specifically, the quinolinone compound as represented by general formula (I) provided in the present invention has an antagonistic effect on a 5-hydroxytryptarnine transporter, has good regulatory activity against a 5-hydroxytryptarnine 1A(5-HT_{1A}) receptor, can be used for treating various central nervous system diseases, and does not cause central or peripheral side effects resulting from the full agonism of the 5-HT_{1A} receptor. In addition, the compound of the present invention has strong activity and high oral bioavailability, has characteristics such as a low effective dosage and low levels of toxicity and few side effects, and is effective in diseases in the field of central nervous systems.

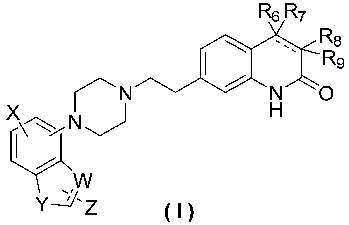

## Description

### Technical Field

The present invention belongs to the field of medicinal chemistry. Specifically, the present invention relates to a class of quinolinone compounds represented by general formula (I), stereoisomers or pharmaceutically acceptable salts thereof, their preparation methods, pharmaceutical compositions, and their use in the preparation of drugs for central nervous system diseases.

### Background

With the rapid development of society, people's pace of life and pressure are increasing, and mental illnesses have become a serious disease affecting human health, bringing severe consequences to patients and their families. Due to factors such as increased suicidal tendencies, lack of medical care, and higher risk of complications, the average lifespan of patients with mental illnesses is shortened. Numerous studies have shown that mental illnesses are related to abnormalities in the function of multiple neurotransmitters and receptors in the central nervous system, such as monoamine neurotransmitters in the brain, especially the dopamine (DA) system and the serotonin (5-HT) system, which are closely related to normal human mental activity. When the DA and 5-HT systems are dysfunctional, it easily leads to the occurrence of various neuropsychiatric diseases, such as schizophrenia, depression, neuropathic pain, mania, anxiety disorders, Parkinson's disease, etc.

The 5-HT transporter is an important target for antidepressants and anxiolytics. Existing SSRls (selective 5-HT reuptake inhibitors) selectively inhibit the 5-HT transporter, increasing the concentration of 5-HT in the synaptic cleft, thereby exerting their antidepressant and anxiolytic effects. The 5-HT_{1A} receptor is the most widely distributed 5-HT receptor subtype in the brain and is involved in many brain-related diseases, including depression, anxiety, and cognitive impairment. Combining 5-HT_{1A} receptor partial agonists (such as buspirone and tanduroone) with 5-HT transporter inhibitors can enhance the antidepressant effect of the 5-HT transporter inhibitors, possibly because the two drugs can synergistically desensitize the 5-HT_{1A} receptor itself. Therefore, compounds possessing both of these effects have promising research prospects. These compounds can act rapidly and their efficacy may be significantly greater than that of 5-HT_{1A} receptor agonists and SSRIs alone. The new drug veprazole, launched in the US in 2011, is a dual-target drug that acts as both a 5-HT_{1A} receptor agonist and SSRI. It takes effect within one week and has no adverse reactions such as sexual dysfunction.

Therefore, finding compounds that can balance and regulate multiple targets, including the 5-HT transporter and 5-HT receptor, is beneficial for better regulating the balance of various neurotransmitters in the brain, modulating the dopamine/5-HT system, and thus better treating diseases of the central nervous system.

In view of this, the present invention is proposed.

### Summary of the Invention

### Objective of the Invention

The objective of the present invention is to provide a class of compounds that antagonize 5-hydroxytryptamine transporters and exhibit good regulatory activity against 5-hydroxytryptamine 1A (5-HT_{1A}) receptors.

One objective of the present invention is to provide a quinolinone compound of general formula (I), its stereoisomers, or a pharmaceutically acceptable salt thereof.

Another objective of the present invention is to provide a method for preparing a quinolinone compound of general formula (I).

A further objective of the present invention is to provide a pharmaceutical composition comprising a therapeutically effective amount of a compound of general formula (I), its stereoisomers, or a pharmaceutically acceptable salt thereof.

A still other objective of the present invention is to provide the use of a quinolinone compound of general formula (I), its stereoisomers, or a pharmaceutically acceptable salt thereof, or the above pharmaceutical composition in the preparation of medicaments for the prevention and/or treatment of central nervous system diseases.

### Technical Solution

According to one aspect of the present invention, it is provided a quinolinone compound represented by general formula (I), its stereoisomers, or a pharmaceutically acceptable salt thereof:

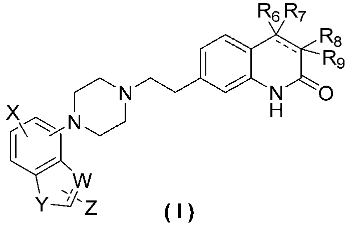

Where:
X on a benzene ring is a halogen, preferably F;
Y is NH, O, or S, preferably O or S;
W is CH, CH₂, or O;
------ represents a single bond or a double bond;
When W is CH₂ or O, the ------ connected to W represents a single bond; when W is CH, the ------ connected to W represents a double bond;
Z on a heterocycle is hydrogen, cyano, amino C1-C6 alkanoyl, or C1-C6 alkyl-substituted amino C1-C6 alkanoyl, preferably, Z is hydrogen, cyano, amino C1-C4 alkanoyl, or C1-C4 alkyl-substituted amino C1-C4 alkanoyl (preferably N-methylcarbamoyl), more preferably, Z is hydrogen, cyano, carbamoyl, or N-methylcarbamoyl;
When the ------ connected to R₆, R₇, R₈, R₉ represents a single bond, R₆, R₇, R₈, and R₉ are each independently selected from the group consisting of hydrogen, deuterium, halogen, methyl, ethyl, hydroxyl, amino, and acetamido, preferably hydrogen, deuterium, halogen, or hydroxyl; or one of R₆ and R₇ and one of R₈ and R₉, along with the two carbon atoms connected with them, form a 3- to 6-membered carbon ring, preferably a 3-membered carbon ring, and the other two substituents are defined as above;
when the ------ connected to R₆, R₇, R₈, R₉ represents a double bond, R₆ and R₈ are each independently selected from the group consisting of hydrogen, deuterium, halogen, methyl, ethyl, hydroxyl, amino, and acetamido, preferably hydrogen, deuterium, or halogen, R₇ and R₉ are absent; Alternatively, R₆ and R₈, along with the two carbon atoms connected with them, form a 3-to 6-membered carbon ring, preferably a 4- to 6-membered carbon ring; R₇ and R₉ are absent; with condition that the following compounds are not included:
   1) 7-(2-(4-(6-fluorobenzothiophene-4-yl)piperazin-1-yl)ethyl)-quinolin-2(1H)-one;
   2) 7-(2-(4-(6-fluorobenzothiophene-4-yl)piperazin-1-yl)ethyl)-3,4-dihydroquinolin-2(1H)-one.

In some preferred embodiments, X is F.

In some preferred embodiments, Y is O or S.

In some preferred embodiments, Z is hydrogen, cyano, amino C1-C4 acyl, or C1-C4 alkyl-substituted amino C1-C4 alkanoyl (preferably N-methylcarbamoyl); preferably, Z is hydrogen, cyano, N-methylcarbamoyl, or carbamoyl.

In some preferred embodiments, the ------ connected to R₆, R₇, R₈, and R₉ represents a single bond, R₆, R₇, R₈, and R₉ are each independently selected from the group consisting of hydrogen, deuterium, halogen, and hydroxyl.

In some preferred embodiments, the ------ connected to R₆, R₇, R₈, and R₉ represents a double bond, R₆ and R₈ are each independently selected from the group consisting of hydrogen, deuterium, halogen, and hydroxyl, and R₇ and R₉ are absent.

In some preferred embodiments, the ------ connected to R₆, R₇, R₈, and R₉ represents a single bond, R₆ and R₈ and the two carbon atoms connected with them form a 3-membered carbon ring; R₇ and R₉ are hydrogen or deuterium.

In some preferred embodiments, X is F, and Y is O.

In some preferred embodiments,
------ represents a single or double bond;
X is a halogen; Y is O or S; W is CH, CH₂, or O; Z is hydrogen, cyano, carbamoyl, or a C1-C6 alkyl-substituted carbamoyl group;
When the ------ connected to R₆, R₇, R₈, or R₉ represents a single bond, R₆, R₇, R₈, and R₉ are each independently selected from the group consisting of hydrogen, deuterium, halogen, methyl, ethyl, hydroxyl, amino, and acetamino; or one of R₆ or R₇ and one of R₈ or R₉, along with the two carbon atoms connected with them, form a 3- to 6-membered carbon ring, and the other two substituents are as defined above;
When the ------ connected to R₆, R₇, R₈, or R₉ represents a double bond, R₆ and R₈ are each independently selected from the group consisting of hydrogen, deuterium, halogen, methyl, ethyl, hydroxyl, amino, and acetamino, preferably hydrogen, deuterium, or halogen, and R₇ and R₉ are absent; or R₆ and R₈, along with the two carbon atoms connected with them, form a 4- to 6-membered carbon ring.

In some preferred embodiments,
------ represents a single or double bond;
X is F; Y is O; W is CH, CH₂, or O; Z is hydrogen, cyano, carbamoyl, or a C1-C6 alkyl-substituted carbamoyl group;
When the ------ connected to R₆, R₇, R₈, R₉ represents a single bond, R₆, R₇, R₈, and R₉ are each independently selected from the group consisting of hydrogen, deuterium, halogen, methyl, ethyl, hydroxyl, amino, and acetamino; or one of R₆ or R₇ and one of R₈ or R₉, along with the two carbon atoms connected with them, form a 3- to 6-membered carbon ring, and the other two substituents are as defined above;
When the ------ connected to R₆, R₇, R₈, R₉ represents a double bond, R₆ and R₈ are each independently selected from the group consisting of hydrogen, deuterium, halogen, methyl, ethyl, hydroxyl, amino, and acetamino, preferably hydrogen, deuterium, or halogen, and R₇ and R₉ are absent; or R₆ and R₈, along with the two carbon atoms connected with them, form a 4- to 6-membered carbon ring.

In some embodiments, the quinolinone compound represented by general formula (I) is selected from the following structures:

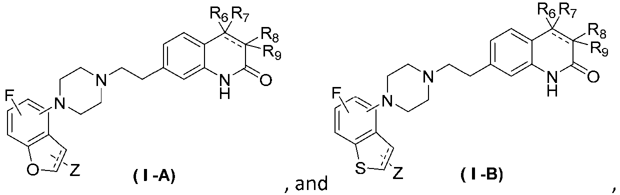

Wherein, ------, Z, R₆, R₇, R₈, and R₉ are as defined above.

In some preferred embodiments, the quinolinone compound represented by general formula (I) is selected from the following structures: 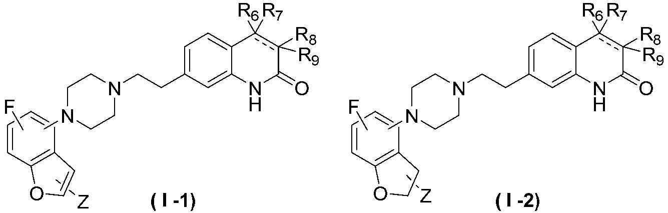 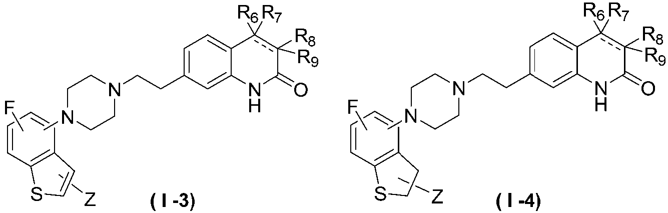 and

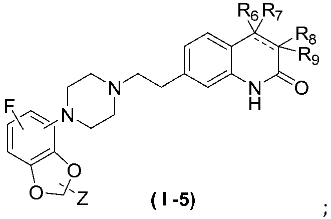

Wherein, ------, Z, R₆, R₇, R₈, and R₉ are as defined above.

In some preferred embodiments, the quinolinone compound represented by general formula (I) is selected from formulas (I-1), (I-2), and (I-5).

In some most preferred embodiments, the quinolinone compound represented by general formula (I) is selected from the following structures: 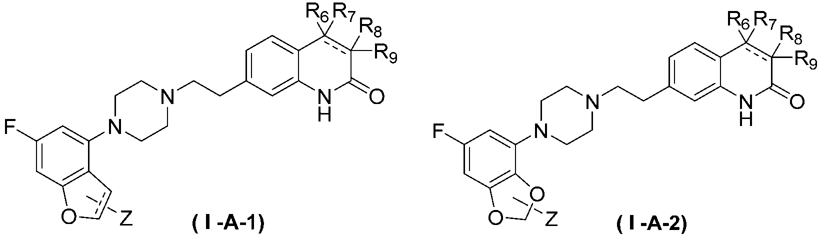 and 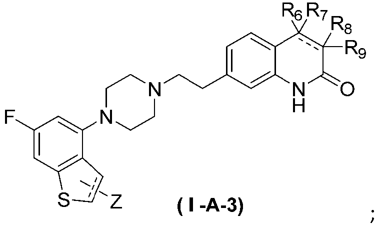 wherein, ------, Z, R₆, R₇, R₈, and R₉ are as defined above.

Among the quinolinone compounds represented by general formula (I), their stereoisomers, or pharmaceutically acceptable salts thereof, the following compounds or pharmaceutically acceptable salts thereof are most preferred:
(1) 7-(2-(4-(6-fluorobenzofuran-4-yl)piperazin-1-yl)ethyl)-3,4-dihydroquinolin-2(1H)-one; 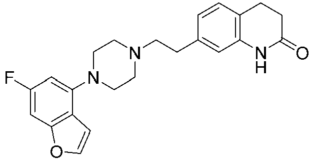
(2) 7-(2-(4-(6-fluoro-2,3-dihydrobenzofuran-4-yl)piperazin-1-yl)ethyl)-3,4-dihydroquinolin-2(1H)-one; 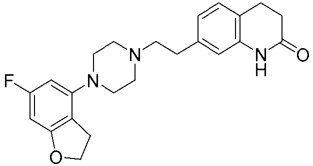
(3) 7 -(2-(4-(5-fluorobenzofuran-7-yl)piperazin-1-yl)ethyl)quinolin-2(1H)-one; 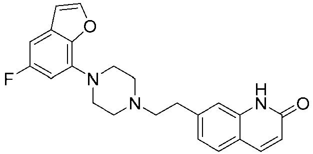
(4) 7-(2-(4-(5-fluoro-2,3-dihydrobenzofuran-7-yl)piperazin-1-yl)ethyl)quinolin-2(1H)-one; 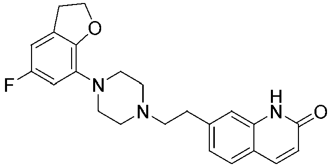
(5) 7-(2-(4-(5-fluoro-2,3-dihydrobenzofuran-7-yl)piperazin-1-yl)ethyl)-3,4-dihydroquinolin-2(1H)-one; 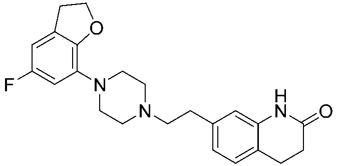
(6) 7-(2-(4-(5-fluorobenzofuran-7-yl)piperazin-1-yl)ethyl)-3,4-dihydroquinolin-2(1H)-one; 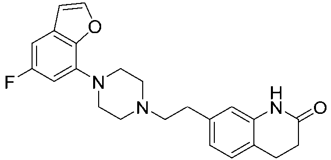
(7) 7-(2-(4-(6-fluoro-2,3-dihydrobenzofuran-4-yl)piperazin-1-yl)ethyl)quinolin-2(1H)-one; 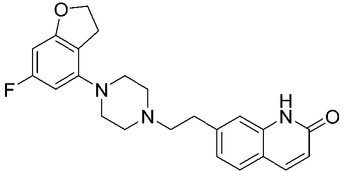
(8) 7-(2-(4-(6-fluorobenzofuran-4-yl)piperazin-1-yl)ethyl)quinolin-2(1H)-one; 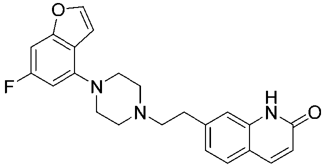
(9) 5-(2-(4-(6-fluorobenzo[b]thiophene-4-yl)piperazin-1-yl)ethyl)-1,1a,3,7b-tetrahydro-2H-cyclopropy l[*c*]quinolin-2-one; 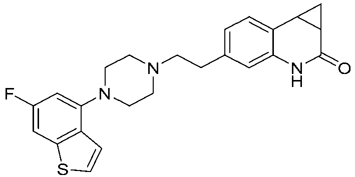
(10) 5-(2-(4-(5-fluorobenzo[b]thiophene-7-yl)piperazin-1-yl)ethyl)-1,1a,3,7b-tetrahydro-2H-cyclopropy l[*c*]quinolin-2-one; 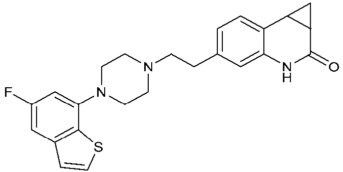
(11) 7-(2-(4-(5-fluorobenzo[b]thiophene-4-yl)piperazin-1-yl)ethyl)-3,4-dihydroquinolin-2(1H)-one; 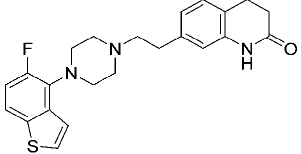
(12) 7-(2-(4-(7-fluorobenzo[*b*]thiophene-4-yl)piperazin-1-yl)ethyl)-3,4-dihydroquinolin-2(1H)-one; 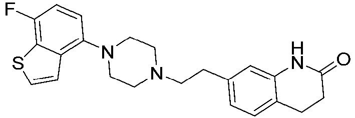
(13) 7-(2-(4-(4-fluorobenzo[*b*]thiophene-7-yl)piperazin-1-yl)ethyl)-3,4-dihydroquinolin-2(1H)-one; 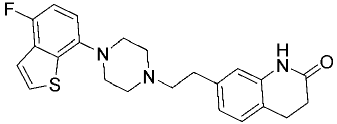
(14) 7-(2-(4-(6-fluorobenzo[*b*]thiophene-7-yl)piperazin-1-yl)ethyl)-3,4-dihydroquinolin-2(1H)-one; 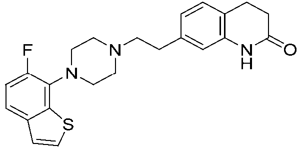
(15) 7 -(2-(4-(5-fluorobenzo[*b*]thiophene-7-yl)piperazin-1-yl)ethyl)-3,4-dihydroquinolin-2(1H)-one; 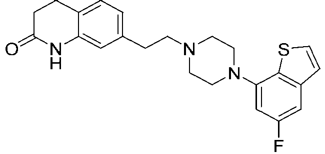
(16) 7-(2-(4-(5-fluorobenzo[*b*]thiophene-7-yl)piperazin-1-yl)ethyl)quinolin-2(1H)-one; 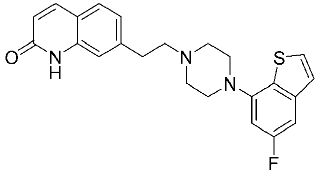
(17) 7-(2-(4-(6-fluorobenzofuran-4-yl)piperazin-1-yl)ethyl)-4-hydroxy-3,4-dihydroquinolin-2(1H)-one; 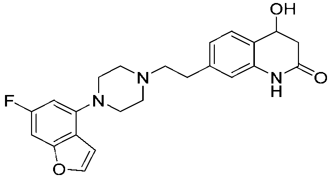
(18) 7-(2-(4-(6-fluoro-2,3-dihydrobenzofuran-4-yl)piperazin-1-yl)ethyl)-4-hydroxy-3,4-dihydroquinolin-2(1H )-one; 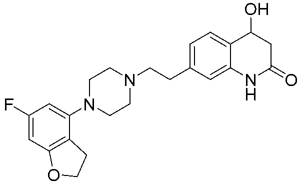
(19) 7-(2-(4-(6-fluorobenzofuran-4-yl)piperazin-1-yl)ethyl)-3,4-dihydroquinolin-2(1H)-one-3,4-d2; 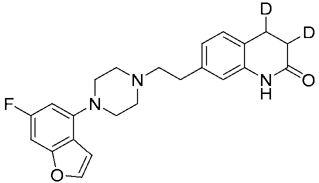
(20) 7-(2-(4-(6-fluorobenzofuran-4-yl)piperazin-1-yl)ethyl)-3,4-dihydroquinolin-2(1H)-one- 3,3-d2; 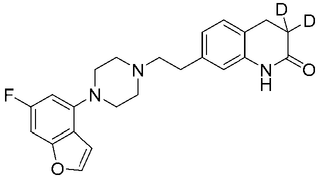
(21) 7-(2-(4-(6-fluoro-2,3-dihydrobenzofuran-4-yl)piperazin-1-yl)ethyl)-3,4-dihydroquinolin-2(1H)-one-3,4-d2; 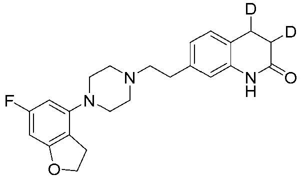
(22)7-(2-(4-(6-fluoro-2,3-dihydrobenzofuran-4-yl)piperazin-1-yl)ethyl)-3,4-dihydroquinolin-2(1H)-one-3,3-d2; 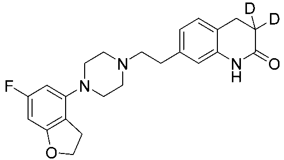
(23) 6-fluoro-4-(4-(2-(2-oxo-1,2, 3,4-tetrahydroquinolin-7-yl)ethyl)piperazin-1-yl)benzo[b]thiophene-2-carboxamide; 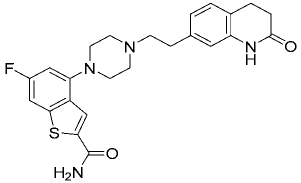
(24) 6-fluoro-4-(4-(2-(2-oxo-1,2-dihydroquinolin-7-yl)ethyl)piperazin-1-yl)benzo[*b*]thiophene-2-carbox amide; 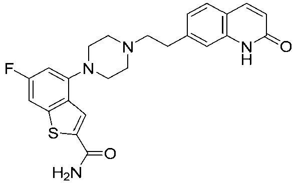
(25) 6-fluoro-4-(4-(2-(2-oxo-1,2,3,4-tetrahydroquinolin-7-yl)ethyl)piperazin-1-yl)benzofuran-2-carboxa mide; 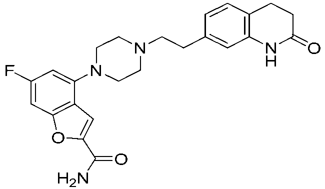
(26) 6-fluoro-4-(4-(2-(2-oxo-1,2-dihydroquinolin-7-yl)ethyl)piperazin-1-yl)benzofuran-2-carboxamide; 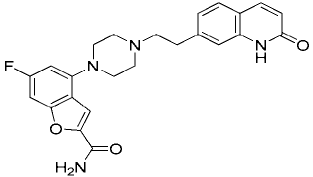
(27) 6-fluoro-N-methyl-4-(4-(2-(2-oxo-1,2,3,4-tetrahydroquinolin-7-yl)ethyl)piperazin-1-yl)benzo[*b*]thi ophene-2-carboxamide; 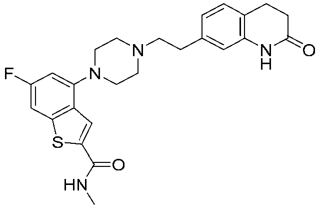
(28) 7-(2-(4-(5-fluorobenzo[*b*]thiophene-4-yl)piperazin-1-yl)ethyl)quinolin-2(1H)-one; 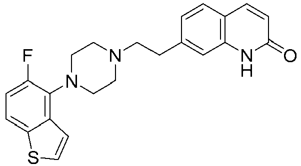
(29) 7-(2-(4-(7-fluorobenzo[*b*]thiophene-4-yl)piperazin-1-yl)ethyl)-quinolin-2(1H)-one; 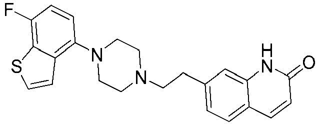
(30) 7-(2-(4-(4-fluorobenzo[*b*]thiophene-7-yl)piperazin-1-yl)ethyl)quinolin-2(1H)-one; 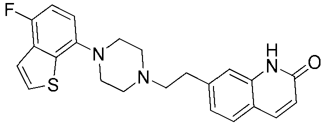
(31) 7-(2-(4-(6-fluorobenzo[*b*]thiophene-7-yl)piperazin-1-yl)ethyl)quinolin-2(1H)-one; 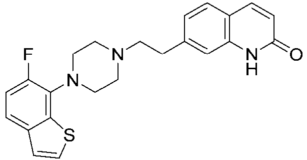
(32) 6-fluoro-N-methyl-4-(4-(2-(2-oxo-1,2-dihydroquinolin-7-yl)ethyl)piperazin-1-yl)benzo[b]thiophen e-2-carboxamide; 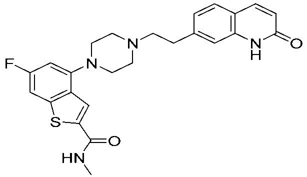
(33) 5-fluoro-7-(4-(2-(2-oxo-1,2,3,4-tetrahydroquinolin-7-yl)ethyl)piperazin-1-yl)benzo[b]thiophene-2-carboxamide; 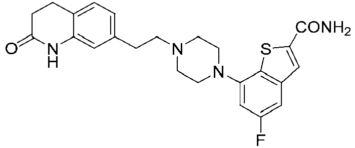
(34) 5-fluoro-7-(4-(2-(2-oxo-1,2-dihydroquinolin-7-yl)ethyl)piperazin-1-yl)benzo[*b*]thiophene-2-carbox amide; 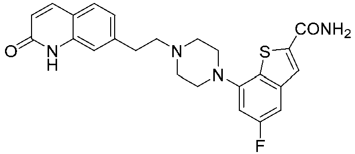
(35) 5-fluoro-7-(4-(2-(2-oxo-1,2,3,4-tetrahydroquinolin-7-yl)ethyl)piperazin-1-yl)benzofuran-2-carboxa mide; 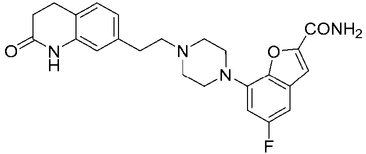
(36) 5-fluoro-7-(4-(2-(2-oxo-1,2-dihydroquinolin-7-yl)ethyl)piperazin-1-yl)benzofuran-2-carboxamide; 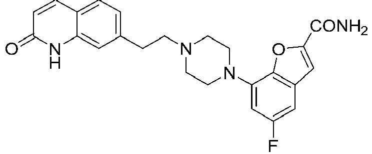
(37) 5-fluoro-7-(4-(2-(2-oxo-1,2,3,4-tetrahydroquinolin-7-yl)ethyl)piperazin-1-yl)benzo[b]thiophene-2-carbonitrile; 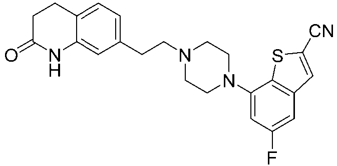
(38) 5-fluoro-7-(4-(2-(2-oxo-1,2-dihydroquinolin-7-yl)ethyl)piperazin-1-yl)benzo[b]thiophene-2-carbon itrile; 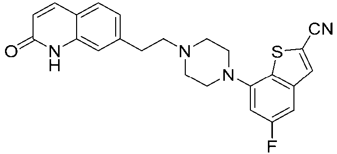
(39) 7-(2-(4-(6-fluorobenzo[d][1,3]dioxol-4-yl)piperazin-1-yl)ethyl)-3,4-dihydroquinolin-2(1H)-one; 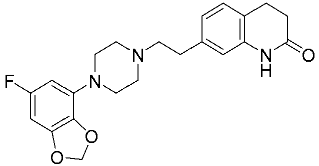
(40) 7-(2-(4-(6-fluorobenzo[d][1,3]dioxol-4-yl)piperazin-1-yl)ethyl)quinolin-2(1H)-one; 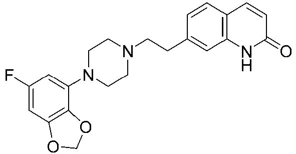
(41) 7-(2-(4-(6-fluorobenzofuran-4-yl)piperazin-1-yl)ethyl)-3,4-dihydroquinolin-2(1H)-one-4,4-d2; 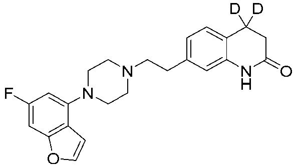
(42) 7-(2-(4-(6-fluoro-2,3-dihydrobenzofuran-4-yl)piperazin-1-yl)ethyl)-3,4-dihydroquinolin-2(1H)-one-4,4-d2; 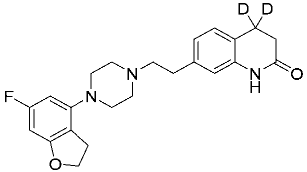
(43) 7-(2-(4-(6-fluorobenzofuran-4-yl)piperazin-1-yl)ethyl)-3,4-dihydroquinolin-2(1H)-one-3,3,4,4-d4; 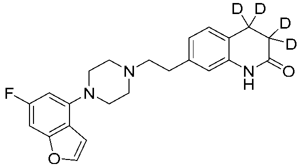
(44) 7-(2-(4-(6-fluoro-2,3-dihydrobenzofuran-4-yl)piperazin-1-yl)ethyl)-3,4-dihydroquinolin-2(1H)-one-3,3,4,4-d4; 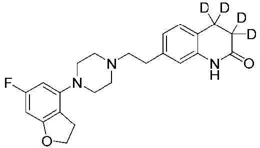
(45) 7-(2-(4-(6-fluorobenzo[*b*]thiophene-4-yl)piperazin-1-yl)ethyl)-4-hydroxy-3,4-dihydroquinolin-2(1H )-one; 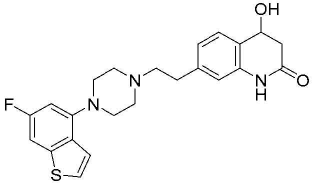
(45-1)R-7-(2-(4-(6-fluorobenzo[*b*]thiophene-4-yl)piperazin-1-yl)ethyl)-4-hydroxy-3,4-dihydroquino lin-2(1H)-one; 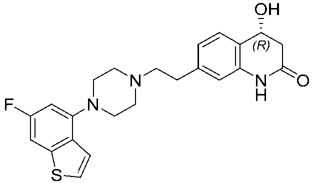
(45-2)S-7-(2-(4-(6-fluorobenzo[*b*]thiophene-4-yl)piperazin-1-yl)ethyl)-4-hydroxy-3,4-dihydroquino lin-2(1H)-one;

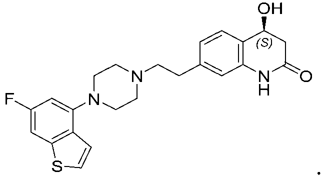

The compounds of the present invention exhibit multi-target activity against 5-HT_{1A} receptors and monoamine transporters, demonstrating superior druggability compared to the compounds in patent application WO2015131856A1. They can be used to treat various central nervous system diseases, particularly depression, bipolar disorder, schizophrenia, anxiety disorders, phobias, autism, Alzheimer's disease, bipolar disorder, hysteria, obsessive-compulsive disorder, and ADHD and the like.

According to a second aspect of the present invention, it is provided a method for preparing a class of quinolinone compounds represented by general formula (I), said preparation method being carried out by one of the following methods 1 and 2.

Method 1:
a compound represented by formula (II) or a salt thereof is reacted with a compound represented by formula (III) or a salt thereof via an N-alkylation reaction with, as shown in reaction scheme 1: 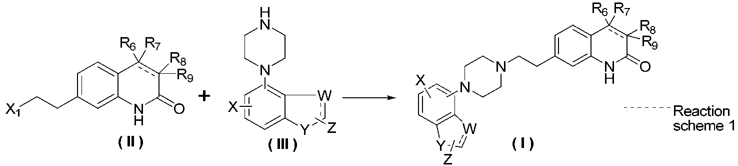
Where, X, Y, W, Z, R₆, R₇, R₈, and R₉ are defined as above;
X₁ represents a leaving group, such as halogen, C1-C6 alkylsulfonyloxy, benzenesulfonyloxy, or naphthalenesulfonyloxy, wherein the aforementioned C1-C6 alkylsulfonyloxy, benzenesulfonyloxy, and naphthalenesulfonyloxy may be optionally further substituted with one or more groups selected from the group consisting of halogen, hydroxyl, amino, nitro, C1-C6 alkyl, C1-C6 alkoxy, and C1-C6 alkanoyl groups;
Preferably, X₁ is halogen, C1-C4 alkylsulfonyloxy, benzenesulfonyloxy, or naphthalenesulfonyloxy, and the aforementioned C1-C4 alkylsulfonyloxy, benzenesulfonyloxy, and naphthalenesulfonyloxy may be optionally further substituted with one or more groups selected from the group consisting of halogen, hydroxyl, amino, nitro, C1-C4 alkyl, C1-C4 alkoxy, and C1-C4 alkanoyl groups;
most preferably, X₁ is chlorine, bromine, methanesulfonyloxy, trifluoromethanesulfonyloxy, benzenesulfonyloxy, naphthalenesulfonyloxy, methylbenzenesulfonyloxy, nitrobenzenesulfonyloxy, aminobenzenesulfonyloxy, chlorobenzenesulfonyloxy, bromobenzenesulfonyloxy, or methoxybenzenesulfonyloxy.

The N-alkylation reaction between the compound of formula (II) or its salt and the compound of formula (III) or its salt is carried out under conditions with or without a solvent, or in a solvent with or without a base. The solvent includes water; ethers, such as dioxane, tetrahydrofuran, diethyl ether, methyl tert-butyl ether, diisopropyl ether, diglyme, dimethoxyethane, etc.; aromatics, such as benzene, toluene, xylene, nitrobenzene, chlorobenzene, etc.; alcohols, such as methanol, ethanol, isopropanol, butanol, tert-butanol, ethylene glycol; ketones, such as acetone, methyl ethyl ketone, 4-methyl-2-pentanone, etc.; amides, such as N,N-dimethylformamide, N,N-dimethylacetamide, 1-methyl-2-pyrrolidone, etc.; halogenated hydrocarbons, such as chloroform, dichloromethane, dichloroethane, carbon tetrachloride; esters, such as ethyl acetate, ethyl formate, methyl acetate, isopropyl acetate; other solvents, such as dimethyl sulfoxide, acetonitrile, etc.; or mixtures of the above solvents.

The base may be selected from the group consisting of inorganic or organic bases. Inorganic bases include alkali metal hydroxides, such as sodium hydroxide, potassium hydroxide, cesium hydroxide, and lithium hydroxide; alkali metal carbonates, such as sodium carbonate, potassium carbonate, cesium carbonate, and lithium carbonate; alkali metal bicarbonates, such as sodium bicarbonate, potassium bicarbonate, and lithium bicarbonate; alkali metals, such as potassium and sodium; and others, such as sodium amino, potassium amino, sodium hydride, and potassium hydride. Organic bases include sodium methoxide, sodium ethoxide, potassium methoxide, potassium ethoxide, sodium acetate, triethylamine, pyridine, diisopropylamine, diisopropylethylamine, tripropylamine, diethylamine, pyrimidine, quinoline, piperidine, piperazine, imidazole, dimethylaminopyridine, trimethylamine, N-ethyldiisopropylamine, N-methylmorpholine, dimethylaniline, 1,8-diazabicyclo[5.4.0]undec-7-ene (DBU), 1,5-diazabicyclo[4.3.0]non-5-ene (DBN), 1,4-diazabicyclo[2.2.2]octane (DABCO). These bases can be used alone or in combination of two or more.

If necessary, alkali metal iodides such as potassium iodide and sodium iodide can be added as reaction promoters to carry out the above reaction.

The above reaction is usually carried out betwee room temperature to 200°C, preferably at room temperature to 150°C, and are usually completed in about 1 to 30 hours, preferably in 5 to 20 hours.

Method 2:
a compound shown in formula (IV) or its salt is reacted with a compound shown in formula (V) or its salt via a coupling reaction, as shown in reaction scheme 2: 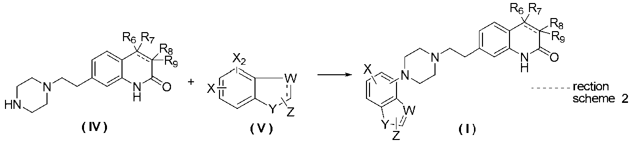
Wherein, ------, X, Y, W, Z, R₆, R₇, R₈, and R₉ are as defined above;
X₂ represents a halogen or trifluoromethanesulfonyloxy group, preferably bromine, iodine, chlorine, or trifluoromethanesulfonyloxy;
The coupling reaction is carried out in the presence of a palladium catalyst and a base, with or without a solvent.

The palladium catalyst is selected from the group consisting of one or more of the following: palladium acetate (Pd(OAc)₂), bis(triphenylphosphine)palladium dichloride ((Ph₃P)₂PdCl₂), bis(benzonitrile)palladium chloride ((PhCN)₂PdCl₂), tetra(triphenylphosphine)palladium (Pd(PPh₃)₄), bis(triphenylphosphine)palladium acetate ((Ph₃P)₂Pd(OAc)₂), 1,2-bis(diphenylphosphine)ethanepalladium dichloride ((PdCl₂(dppe)₂), bis(1,2-bis(diphenylphosphine)ethane)palladium (Pd(dppe)₂), bis(dibenzylideneacetone)palladium (Pd(dba)₂), tris(dibenzylideneacetone)dipalladium (Pd₂(dba)₃), [1,3-bis(diphenylphosphine)propane]palladium dichloride (PdCl₂(dippp)) and [1,1'-bis(diphenylphosphine)ferrocene]palladium dichloride (Pd(dppf)Cl₂).

The base is selected from the group consisting of one or more of the following: sodium bis(trimethylsilyl)amide, potassium tert-butoxide, sodium tert-butoxide, cesium carbonate, potassium phosphate, sodium phosphate, sodium methoxide, sodium ethoxide, potassium hydroxide, sodium hydroxide, potassium fluoride, sodium fluoride, tetrabutylammonium fluoride (TBAF), sodium acetate, potassium acetate, cesium carbonate, potassium carbonate, and sodium carbonate.

The solvent is not particularly limited, as long as it does not interfere with the reaction, and it includes water; ethers, such as dioxane and tetrahydrofuran, etc.; aromatic hydrocarbons, such as toluene and xylene, etc.; alcohols, such as tert-butanol, etc.; ketones, such as acetone, etc.; amides, such as N,N-dimethylformamide, etc.; other solvents, such as dimethyl sulfoxide and acetonitrile, etc.; or mixtures of the above solvents.

If necessary, a suitable ligand can be added as a reaction promoter to carry out the above reaction. Suitable ligands include 2,2'-diphenylphosphino-1,1'-binaphthyl (BINAP), Tri-tert-butylphosphine (P(t-Bu)₃), 1,1'-di-(diphenylphosphino)ferrocene (dppf), 2-dicyclohexylphosphine-2,4,6-triisopropylbiphenyl (x-phos), 4,5-bis(diphenylphosphine)-9,9-dimethyloxanthracene (Xantphos), tri-tert-butylphosphine tetrafluoroborate, and tris(2-methylphenyl)phosphine (P(o-tolyl)₃).

Compounds of formulas (II), (III), (IV), and (V) are commercially available compounds or prepared by methods known in the art or by synthetic methods for preparing similar compounds.

The starting compounds used in the above reaction schemes can be their suitable salts, including their alkali metal salts and alkaline earth metal salts, such as sodium, potassium, calcium, and magnesium salts; organic base salts, such as pyridinium salts and triethylamine salts; inorganic acid salts, such as hydrochloride, hydrobromide, hydroiodide, sulfate, nitrate, and phosphate, etc.; and organic acid salts, such as formate, acetate, propionate, glycolate, oxalate, malonate, succinate, fumarate, maleate, lactate, malate, citrate, tartrate, picrate, glutamate, methanesulfonate, and benzenesulfonate, etc.

Furthermore, the starting compounds used in the above reaction schemes can include their solvates, such as hydrates and alcohols, etc.

The quinolinone compounds represented by general formula (I) and their stereoisomers of the present invention also include their solvate forms, such as hydrates and alcohols, etc., and these solvates are included within the scope of the present invention.

Pharmaceutically acceptable salts of the quinolinone compounds represented by general formula (I) and their stereoisomers of the present invention refer to non-toxic addition salt forms with therapeutic activities of the quinolinone compounds represented by general formula (I) or their stereoisomers, which is converted therefrom by treating the same with a suitable acid. Examples of such salts include hydrochloride, hydrobromide, hydroiodide, sulfate or bisulfate, nitrate, phosphate or acid phosphate, perchlorate, formate, acetate, trifluoroacetate, propionate, pyruvate, glycolate, oxalate, malonate, succinate, glutarate, maleate, fumarate, lactate, malate, citrate, tartrate, picrate, glutamate, benzoate, methanesulfonate, ethanesulfonate, benzenesulfonate, p-toluenesulfonate, salicylate, ascorbate, camphorate or camphorsulfonate, etc. Conversely, the salt form can be converted to a free base form by alkali treatment.

The term "pharmaceutically acceptable salt" as used above also includes their solvates, and said solvates are included within the scope of the present invention. Examples of solvates include, for example, hydrates and alcohols.

The target compounds obtained from each reaction sheme can be separated and purified from the reaction mixture by methods below: separation of a crude product by cooling the reaction mixture, and then subjecting it to filtration, extraction, or concentration and the like, followed by purification by conventional methods such as column chromatography, pulping, or recrystallization.

According to a third aspect of the invention, it is also provided a pharmaceutical composition, comprising a therapeutically effective amount of the quinolinone compound represented by general formula (I), its stereoisomer or a pharmaceutically acceptable salt thereof as mentioned above, and optionally a pharmaceutically acceptable carrier.

According to a fourth aspect of the invention, it is further provided a method for preparing a pharmaceutical composition, comprising mixing the quinolinone compound represented by general formula (I), its stereoisomer or a pharmaceutically acceptable salt thereof as mentioned above with a pharmaceutically acceptable carrier.

In the pharmaceutical compositions of the present invention, various pharmaceutical formulations can be selected according to the therapeutic purpose, including but not limited to: tablets, pills, capsules, granules, suspensions, solutions, creams, ointments, powders, suppositories, aerosols, and injections (e.g., lipid-soluble or oil-soluble injections) and the like.

According to a fifth aspect of the invention, it is further provided the use of the quinolinone compound of general formula (I), its stereoisomers, or pharmaceutically acceptable salts thereof as mentioned above, or the above pharmaceutical composition, in the preparation of a medicament for the prevention and/or treatment of central nervous system diseases.

According to a sixth aspect of the invention, it is further provided a method for treating and/or preventing central nervous system diseases, comprising administering to a human or animal the quinolinone compound of general formula (I), its stereoisomers, or pharmaceutically acceptable salts thereof as mentioned above.

The aforementioned central nervous system diseases are selected from the group consisting of: schizophrenia; affective disorders; mental disorders; mood disorders; type I bipolar disorder; type II bipolar disorder; depression; dysphoric disorder; cyclothymic disorder; panic disorder; obsessive-compulsive disorder; impulsivity disorder; post-traumatic stress disorder; anxiety disorder; hysteria; anorexia nervosa; sleep disorders; adjustment disorders; cognitive impairment; autism; nervous headache; mania; Parkinson's disease; Huntington's disease; various dementias; memory impairment; ADHD; attention deficit/hyperactivity disorder and tic disorders, etc.

Preferably, the aforementioned central nervous system diseases are selected from the group consisting of: refractory, difficult-to-treat, or chronic schizophrenia; endogenous depression; major depressive disorder; refractory depression; panic attacks; social phobia; acute stress disorder; and Alzheimer's disease.

The compounds of the present invention have the following **beneficial effects:**
1) The compounds of the present invention have a good inhibitory effect on the 5-HT transporter and can be used to treat various central nervous system diseases.
2) The compounds of the present invention exhibit good regulatory effects on 5-HT_{1A} receptors, with EC₅₀ or IC₅₀ levels reaching 0.1-10 nM. They can be used to treat various central nervous system diseases without causing central or peripheral side effects resulting from complete 5-HT_{1A} receptor activation.
3) The compounds of the present invention are not only highly active but also orally effective (i.e., high bioavailability), characterized by low effective dose and minimal toxicity. They are effective for diseases of the central nervous system, particularly for major depressive disorder (MDD), anxiety disorders, negative symptoms of schizophrenia, cognitive impairment, Parkinson's disease, and ADHD.

In summary, compared with compounds disclosed in the prior art, the compounds of the present invention have advantages such as multi-target action, lower effective dose, fewer toxic side effects, better safety and tolerability, and good overall druggability, thus showing promising clinical application prospects.

### Description of Drawings

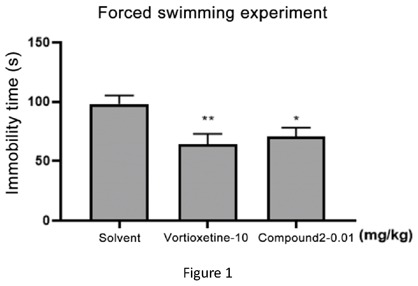

Figure 1 shows the results of the forced swimming experiment of Test Example 5, where * indicates P<0.05, ** indicates P<0.01 (tested using one-way ANOVA).

### Detailed Embodiments

The following preparation examples, examples, and pharmacological activity test examples further illustrate the present invention, but do not limit the scope of the invention.

Unless otherwise specified, the raw materials, reagents, methods, etc. used in the examples and experimental examples are all conventional raw materials, reagents, and methods in the art.

### Preparation Examples

### Example 1 Preparation of 7-(2-(4-(6-fluorobenzofuran-4-yl)piperazin-1-yl)ethyl)-3,4-dihydroquinolin-2(1H)-one

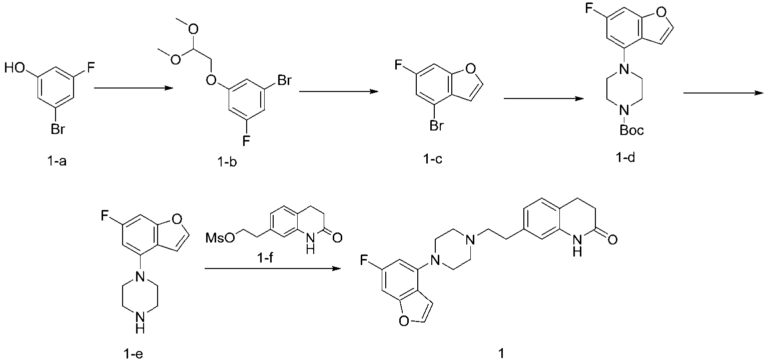

### (1) Synthesis of compound 1-b:

Under nitrogen protection, 3-fluoro-5-bromophenol (6 g, 1 eq), 2-bromo-1,1-dimethoxyethane (7.44 g, 1.4 eq) and potassium carbonate (6.51 g, 1.5 eq) were dissolved in 50 ml of DMF. The mixture was heated to 100°C and stirred overnight. The reaction solution was poured into water, extracted three times with ethyl acetate (EA), and the organic phase was washed twice with saturated sodium bicarbonate solution. The organic phase was dried and concentrated to give compound 1-b, 10.7 g, a brownish-red oily substance, with a yield >99%.

### (2) Synthesis of compound 1-c:

Under nitrogen protection, 1-b (10.7 g, 1 eq) and polyphosphoric acid (PPA) (38 g, 3 eq) were dissolved in toluene and heated under reflux at 110°C for 2 h. The reaction solution was poured into water, and the organic phase was washed 2-3 times with water, and then 1-2 times with saturated sodium bicarbonate solution. The organic phase was dried and concentrated, and purified by column chromatography to give compound 1-c, 4.734 g, an oily substance, with a yield of 57%.

### (3) Synthesis of Compound 1-d

Under nitrogen protection, 1-c (4.734 g, 1 eq), tris(dibenzylindeneacetone)dipalladium (Pd₂(dba)₃) (1.8 g, 0.1 eq), potassium tert-butoxide (6.16 g, 2.5 eq), 1,1'-binaphthyl-2,2'-bis(diphenylphosphine) (BINAP) (1.25 g, 0.1 eq), and 1-Boc-piperazine (4.9 g, 1.2 eq) were dissolved in toluene. The mixture was refluxed at 100 °C, and the reaction was monitored by TLC. After the reaction was complete, the mixture was filtered, concentrated, and purified by column chromatography to give compound 1-d, 1.604 g, a yellow oily substance, with a yield of 22%.

### (4) Synthesis of Compound 1-e

1-d (800 mg, 1 eq) was dissolved in 2 ml of ethanol, and 5 ml of 2 M hydrogen chloride ethanol solution was added. The mixture was stirred overnight at room temperature. The solvent was removed by concentration under reduced pressure, then isopropyl acetate was added for pulping. The resultant was filtered, and the filter cake was dried to give compound 1-e, 600 mg, a yellow solid (hydrochloride form), with a yield of 93%.

### (5) Synthesis of Compound 1:

1-e (600 mg, 1 eq), 1-f (823 mg, 1.3 eq), sodium bicarbonate (1 g, 5.0 eq), sodium iodide (360 mg, 1 eq), and DMF (6 mL) were added to a 50 mL single-necked flask, and the mixture was reacted overnight at 80 °C under nitrogen protection. The mixture was added with water and stirred. The mixture was extracted twice with EA, the organic phase was dried, concentrated under reduced pressure, and purified by column chromatography to give a product, 710 mg.

¹H NMR (400 MHz, DMSO-*d*₆) δ 10.07 (s, 1H), 7.96 (d, J = 2.3 Hz, 1H), 7.13- 7.08 (m, 2H), 7.04 (d, J = 2.2 Hz, 1H), 6.85 (dd, J = 7.6, 1.7 Hz, 1H), 6.79 (d, J = 1.7 Hz, 1H), 6.59 (dd, J = 12.5, 2.2 Hz, 1H), 3.24 (t, J = 4.7 Hz, 4H), 2.87 (t, J = 7.5 Hz, 2H), 2.77 - 2.68 (m, 6H), 2.63 - 2.59 (m, 2H), 2.48 (dd, J = 8.5, 6.5 Hz, 2H). ESI-MS (*m*/*z*): 394.3 [M+H]⁺.

### Example 2 Preparation of 7-(2-(4-(6-fluoro-2,3-dihydrobenzofuran-4-yl)piperazin-1-yl)ethyl)-3,4-dihydroquinolin-2(1H)-on e

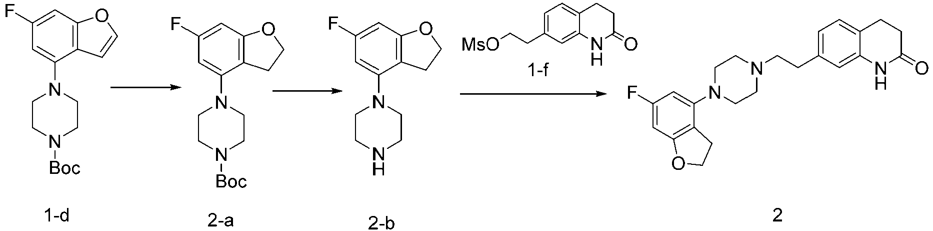

### (1) Synthesis of compound 2-a

1-d (0.437 g, 1 eq) was dissolved in 5 ml of methanol, and 5% palladium on carbon (40 mg, 10% wt) was added. The mixture was pressurized with hydrogen to 2.5 MPa and stirred overnight at 40 °C. the mixture was filted, and concentrated, purified by column chromatography to give compound 2-a, 302 mg, a pale yellow solid, with a yield of 68%.

### (2) Synthesis of Compound 2-b

2-a (302 mg, 1 eq) was dissolved in 5 mL of ethanol, and 2 M hydrogen chloride ethanol solution (8 mL) was added. The mixture was stirred overnight at room temperature. The resultant was concentrated under reduced pressure to remove the solvent, and then isopropyl acetate was added for pulping. The resultant was filtered, and the filter cake was dried to give compound 2-b, 156 mg, a yellow solid (hydrochloride form), with a yield of 64%.

### (3) Synthesis of Compound 2

2-b (200 mg, 1.0 eq), 1-f (340 mg, 1.5 eq), DMF (6 mL), sodium bicarbonate (378 mg, 5.0 eq), and sodium iodide (140 mg, 1.0 eq) were added to a 25 mL three-necked flask under nitrogen protection. The mixture was heated to 80 °C and stirred overnight. After adding water, the mixture was extracted twice with EA. The organic phases were combined, dried, concentrated under reduced pressure, and purified by column chromatography to give a product, 200 mg.

¹H NMR (400 MHz, DMSO-*d*₆) δ 10.01 (s, 1H), 7.06 (d, J = 7.6 Hz, 1H), 6.78 (dd, J = 7.6, 1.7 Hz, 1H), 6.71 (d, J = 1.7 Hz, 1H), 6.27 - 6.18 (m, 2H), 4.52 (t, J = 8.5 Hz, 2H), 3.08 - 2.99 (m, 6H), 2.82 (t, J = 7.5 Hz, 2H), 2.67 (t, J = 7.7 Hz, 2H), 2.56 - 2.54 (m, 6H), 2.42 (dd, J = 8.5, 6.5 Hz, 2H). ESI-MS (*m*/*z*): 394.3 [M+H]⁺.

### Example 9 Preparation of 5-(2-(4-(6-fluorobenzo[b]thiophene-4-yl)piperazin-1-yl)ethyl)-1,1a,3,7b-tetrahydro-2H-cyclopro pyl[c]quinolin-2-one

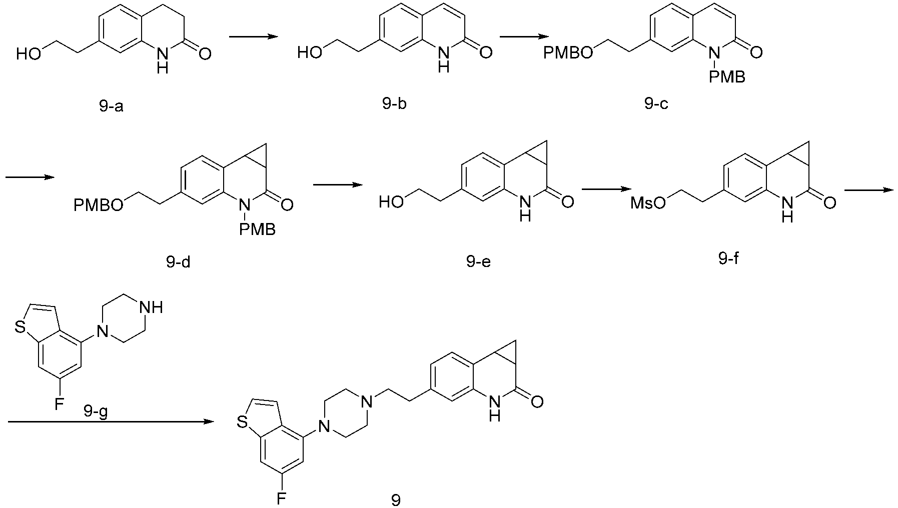

### (1) Preparation of compound 9-b

Compound 9-a (10 g, 1.0 eq), potassium persulfate (28.3 g, 2.0 eq), and copper sulfate pentahydrate (1.3 g, 0.1 eq) were added to acetonitrile (100 mL) and water (100 mL), and the mixture was heated to 80 °C and stirred for 3 hours. The reaction mixture was cooled to room temperature, extracted with methyltetrahydrofuran, and the organic phase was dried, concentrated under reduced pressure to dryness, and stirred with 50 mL of ethanol for 30 min. The mixture was filtered, and the filter cake was dried to give compound 9-b, 3.6 g, a yellow solid.

### (2) Preparation of Compound 9-c

Compound 9-b (3.6 g, 1.0 eq) was added to N,N-dimethylformamide (DMF) (36 mL), and the mixture was cooled to 0°C with stirring under nitrogen protection. NaH (3.0 g, 4.0 eq) was slowly added, and the mixture was stirred at this temperature for 1 hour. Then, p-methoxybenzyl chloride (PMBCI) (12.0 g, 4.0 eq) was slowly added, and the mixture was heated to room temperature for 3 hours. Water was slowly added to the reaction mixture, and the mixture was extracted with ethyl acetate. The organic phase was dried, and the solution was purified by column chromatography to give compound 9-c, 2.4 g, a yellow solid.

### (3) Synthesis of Compound 9-d

Trimethyl sulfoxide (5.1 g, 10.0 eq) was added to tetrahydrofuran (THF) (50 mL), and the mixture was cooled to 0 °C under nitrogen protection. Butyllithium (9.3 mL, 10.0 eq) was slowly added, and the mixture was kept at this temperature for 30 min. Then, compound 9-c (1.0 g, 1.0 eq)/THF (5 mL) was slowly added, and the mixture was heated to 70 °C and reacted for 3 hours. The reaction mixture was then diluted with water, extracted with ethyl acetate, and the organic phase was dried, and concentrated to dryness to give compound 9-d, 1 g, a yellow solid.

### (4) Synthesis of Compound 9-e

Compound 9-d (1.0 g, 1.0 eq) was added to trifluoroacetic acid (10 mL), and the mixture was stirred at 70 °C for 3 hours. The reaction mixture was directly concentrated under reduced pressure to remove the solvent, quenched with amine methanol solution, filtered, and the mother liquor was concentrated and purified by column chromatography to give compound 9-e, 320 mg, an off-white solid.

### (5) Synthesis of Compound 9-f

Compound 9-e (320 mg, 1.0 eq) and triethylamine (TEA) (230 mg, 1.5 eq) were added to dichloromethane (DCM) (3 mL). A solution of methanesulfonic anhydride (320 mg, 1.2 eq)/DCM (2 mL) was slowly added dropwise at 0 °C. After the addition was complete, the mixture was stirred at room temperature for 0.5 hours. The reaction mixture was quenched with water, and 20 mL of n-heptane was added with stirring. A solid precipitated, which was filtered and the filter cake was dried to give compound 9-f, 280 mg, an off-white solid.

### (6) Synthesis of Compound 9

Compound 9-f (100 mg, 1.0 eq), compound 9-g (130 mg, 1.1 eq), and potassium carbonate (120 mg, 2.0 eq) were added to acetonitrile (3 mL). The mixture was heated to 80 °C and stirred for 13 hours. The reaction mixture was quenched with water, and a solid precipitated, which was filtered and the filter cake was dried to give compound 9, 125 mg, an off-white solid.

¹H NMR (400 MHz, DMSO-*d*₆) δ 9.89 (s, 1H), 7.67 (d, J = 5.5 Hz, 1H), 7.51 (d, J = 8.8 Hz, 1H), 7.38 (d, J = 5.6 Hz, 1H), 7.28 (d, J = 7.7 Hz, 1H), 6.90-6.79 (m, 1H), 6.80-6.71 (m, 2H), 3.11 (s, 4H), 2.72-2.68 (m, 6H), 2.59-2.55 (m, 2H), 2.50-2.47(m, 1H), 2.00-1.95 (m, 1H), 1.58-1.53 (m, 1H), 0.48 (q, J = 4.7 Hz, 1H). ESI-MS (m/z):422.1 [M+H]⁺.

### Example 10 Preparation of 5-(2-(4-(5-fluorobenzo[b]thiophene-7-yl)piperazin-1-yl)ethyl)-1,1a,3,7b-tetrahydro-2H-cyclopro pyl[c]quinolin-2-one

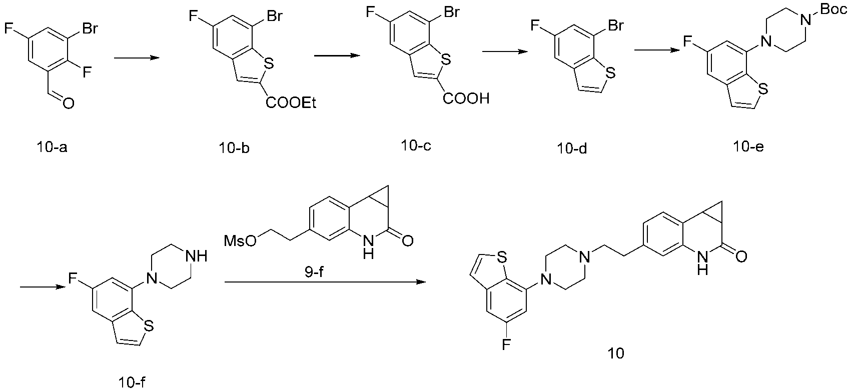

### (1) Synthesis of compound 10-b

Under nitrogen protection, compound 10-a (5.0 g, 1.0 eq), ethyl mercaptoacetate (2.7 g, 1.0 eq), and potassium carbonate (6.2 g, 2.0 eq) were added to DMF (50 mL) and stirred at 60°C for 2 hours. The reaction was quenched with 150 mL of water, precipitating an off-white solid. The solid was filtered, dried, recrystallized with ethanol, filtered again, and the filter cake was dried to give compound 10-b, an off-white solid (4.7 g, yield 69%).

### (2) Synthesis of compound 10-c

Compound 10-b (4.0 g, 1.0 eq) and sodium hydroxide (1.0 g, 2.0 eq) were added to a mixed solvent of THF (50 mL), methanol (15 mL), and water (20 mL). The mixture was stirred at room temperature for 12 hours. The organic phase was concentrated under reduced pressure, and the pH was adjusted to 2-3 with 1 M HCl. A white solid precipitated, which was filtered, and the filter cake was dried to give compound 10-c, an off-white solid (3.4 g, yield 94%).

### (3) Synthesis of Compound 10-d

Under nitrogen protection, compound 10-c (3.4 g, 1.0 eq) and cuprous oxide (5.4 g, 3.0 eq) were added to N,N-dimethylformamide (30 mL), and the mixture was stirred at 145°C for 6 hours. The reaction solution was filtered, purified water was added to the filtrate with stirring at room temperature for crystallization. After filtration, the filter cake was dried to give compound 10-d, 0.8 g, a yellow solid.

### (4) Synthesis of Compound 10-e

Under nitrogen protection, compound 10-d (400 mg, 1.0 eq), N-Boc piperazine (480 mg, 1.5 eq), 1,1'-binaphthyl-2,2'-bis(diphenylphosphine) (BINAP) (108 mg, 0.1 eq), Pd₂(dba)₃ (159 mg, 0.1 eq), and potassium tert-butoxide (388 mg, 2.0 eq) were added to toluene (10 ml), and the mixture was stirred at 100 °C for 12 hours. The mixture was filtered, the filtrate was concentrated under reduced pressure, and the organic phase was purified by column chromatography to give compound 10-e, an off-white solid (220 mg).

### (5) Synthesis of Compound 10-f

Compound 10-e (220 mg, 1.0 eq) was dissolved in 2 ml of ethanol, and 2 M hydrogen chloride ethanol solution (5 ml) was added. The mixture was stirred overnight at room temperature. After concentrating the organic phase, water was added, the pH was adjusted to 7-8. The mixture was extracted with ethyl acetate, dried, and concentrated under reduced pressure to give compound 10-f, 150 mg, a yellow solid.

### (6) Synthesis of Compound 10:

Compound 9-f (100 mg, 1.0 eq), compound 10-f (130 mg, 1.1 eq), and potassium carbonate (120 mg, 2.0 eq) were added to acetonitrile (3 mL), and the mixture was heated to 80 °C and stirred for 13 hours. The reaction solution was quenched with water, and a solid precipitated. The solid was filtered and dried to give an off-white solid product, 50 mg.

¹H NMR (400 MHz, DMSO-*d*₆) δ 9.88 (s, 1H), 7.82 (d, J = 5.4 Hz, 1H), 7.42 (d, J = 5.4 Hz, 1H), 7.35 (dd, J = 9.1, 2.3 Hz, 1H), 7.27 (d, J = 7.7 Hz, 1H), 6.85 - 6.81 (m, 2H), 6.73 (d, J = 1.6 Hz, 1H), 3.19 (t, J = 4.7 Hz, 4H), 2.71-2.64 (m, 6H), 2.58-2.54 (m, 2H), 2.50 - 2.47 (m, 1H), 2.00-1.94 (m, 1H), 1.58-1.52 (m, 1H), 0.48 (q, J = 4.7 Hz, 1H). ESI-MS (m/z):422.2 [M+H]⁺.

The table below shows the compounds in Examples 3-8 and 11-45. These compounds could be prepared using the same methods as those in the examples above, except that the starting materials and intermediates corresponding to the final products were used.

For the compound in Example 45, isomers 45-1 and 45-2 were chirally separated by HPLC under the following chromatographic conditions:

| Chromatographic column | CHIRALPAK IC 4.6×250mm,5µm |
|---|---|
| Mobile phase A | n-Hexane |
| Mobile phase B | 0.1% Diethylamine(isopropanol as diluent) |
| Isocratic elution | Mobile phase A:Mobile phase B =80:20 |
| Detection wavelength | 210 nm |
| Column temperature | 35°C |
| Flow rate | 1.0 ml/min |
| Injection volume | 5µl |
| Time | 40min |
| Sample concentration | 1.0 mg/ml(ethanol as a diluent) |

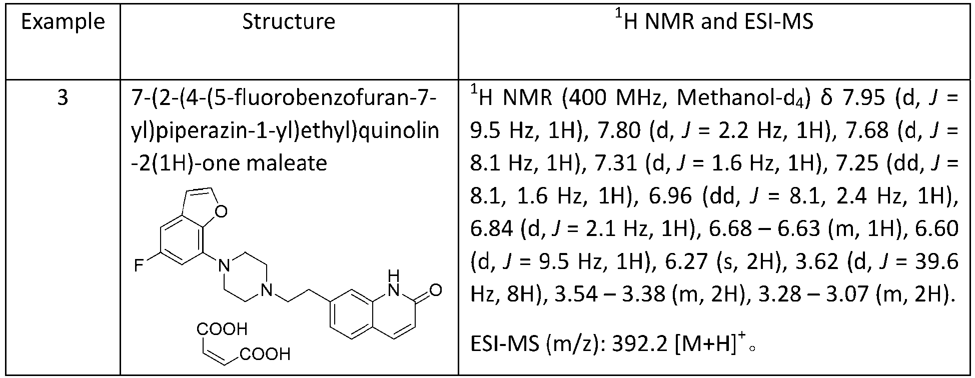

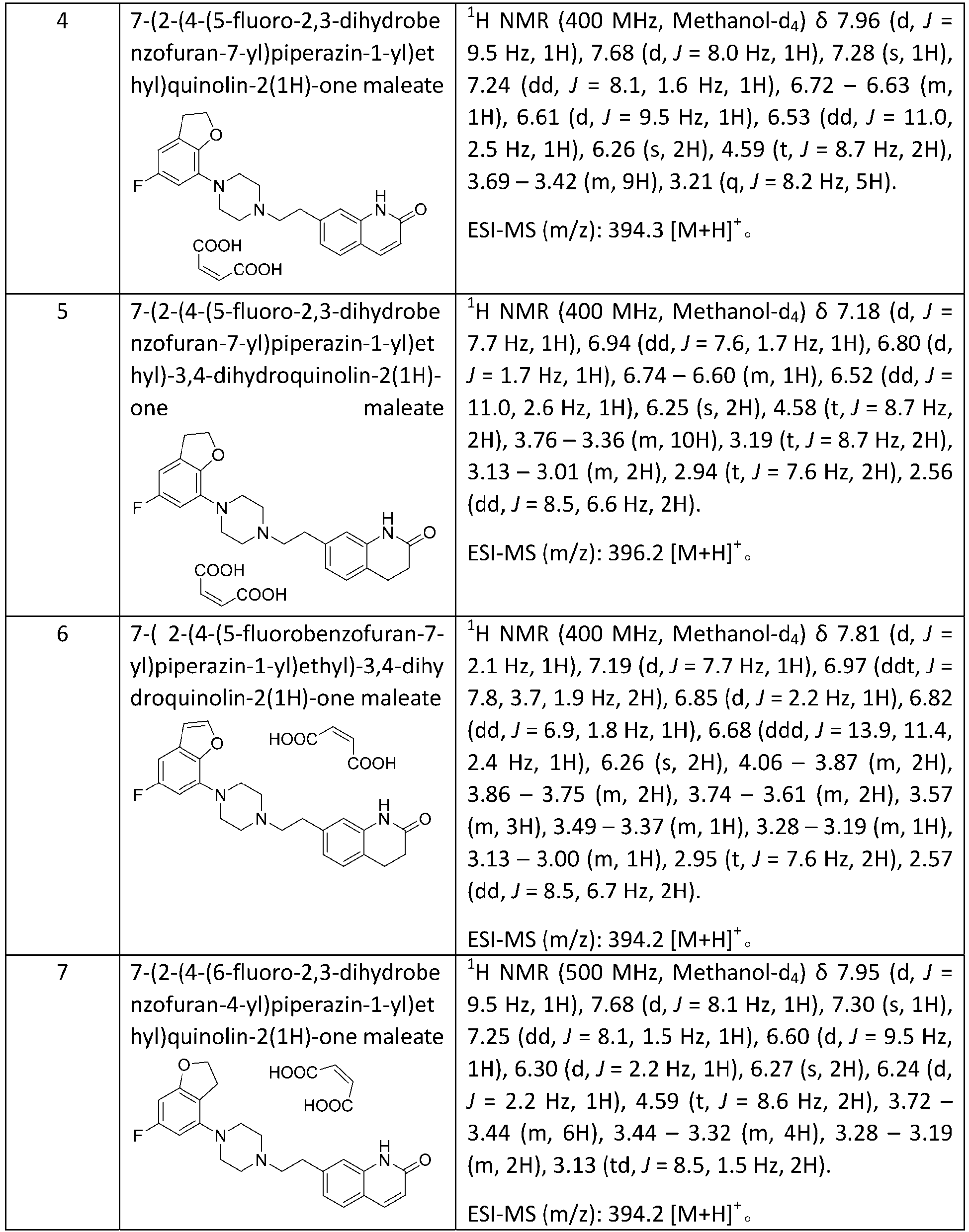

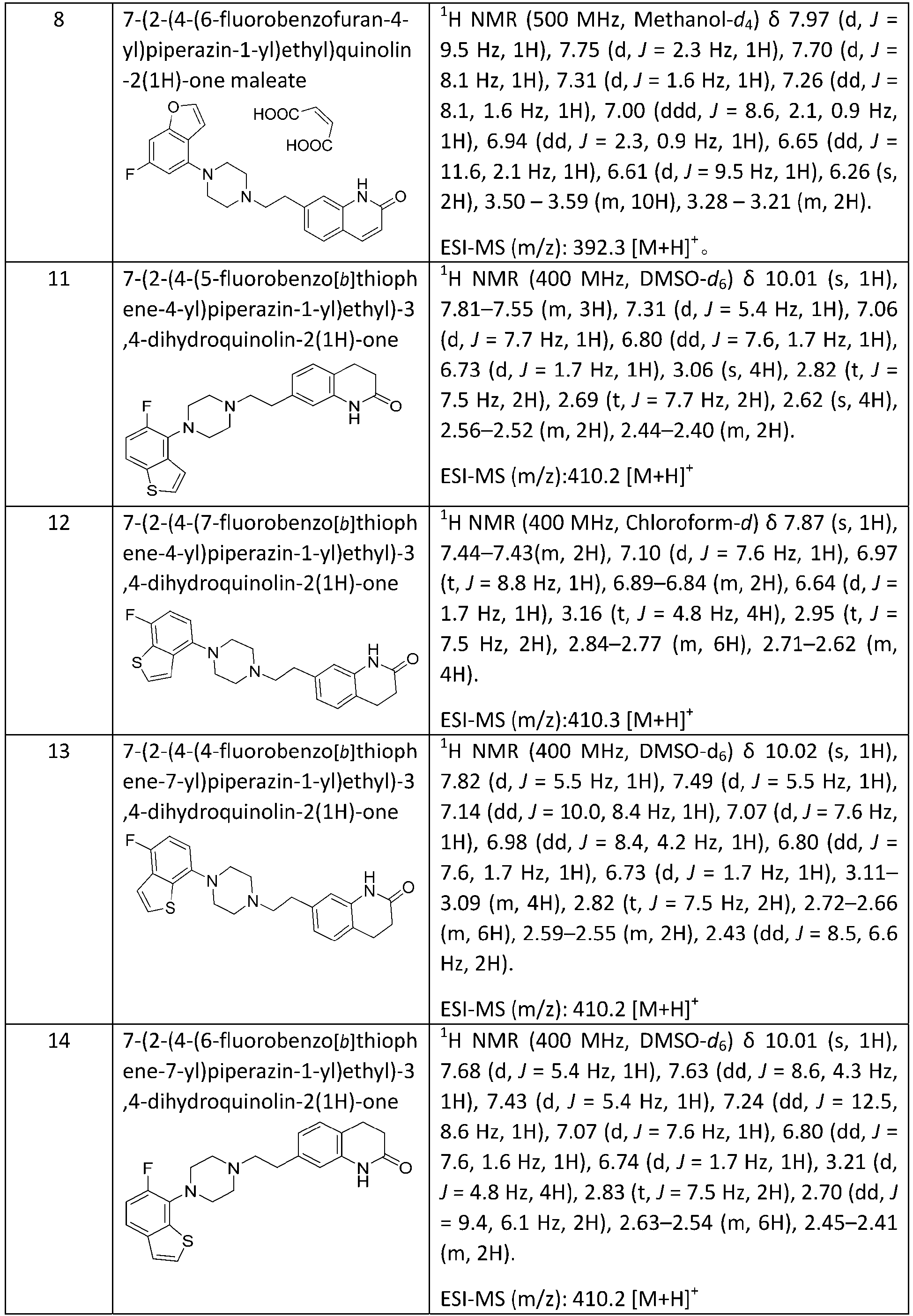

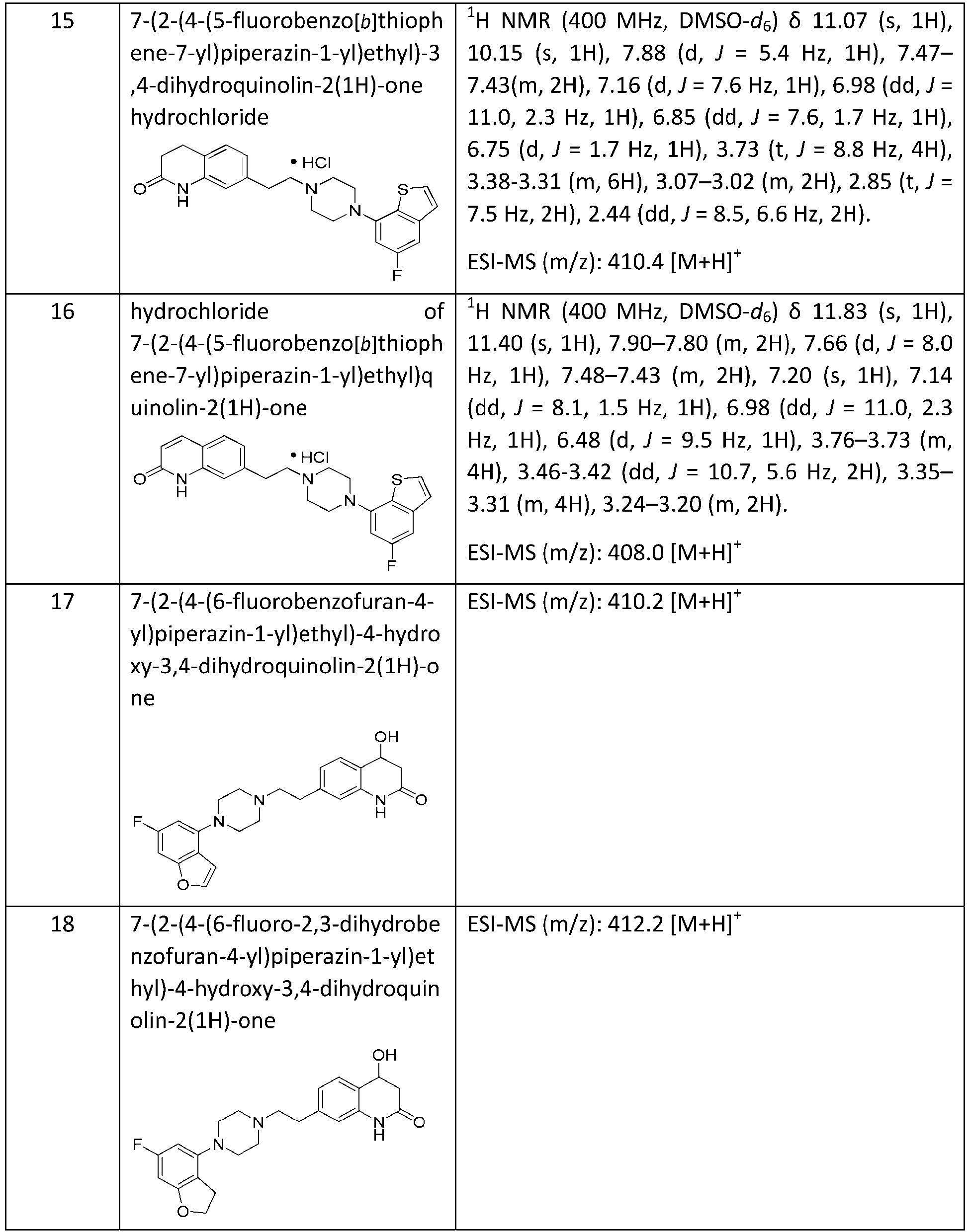

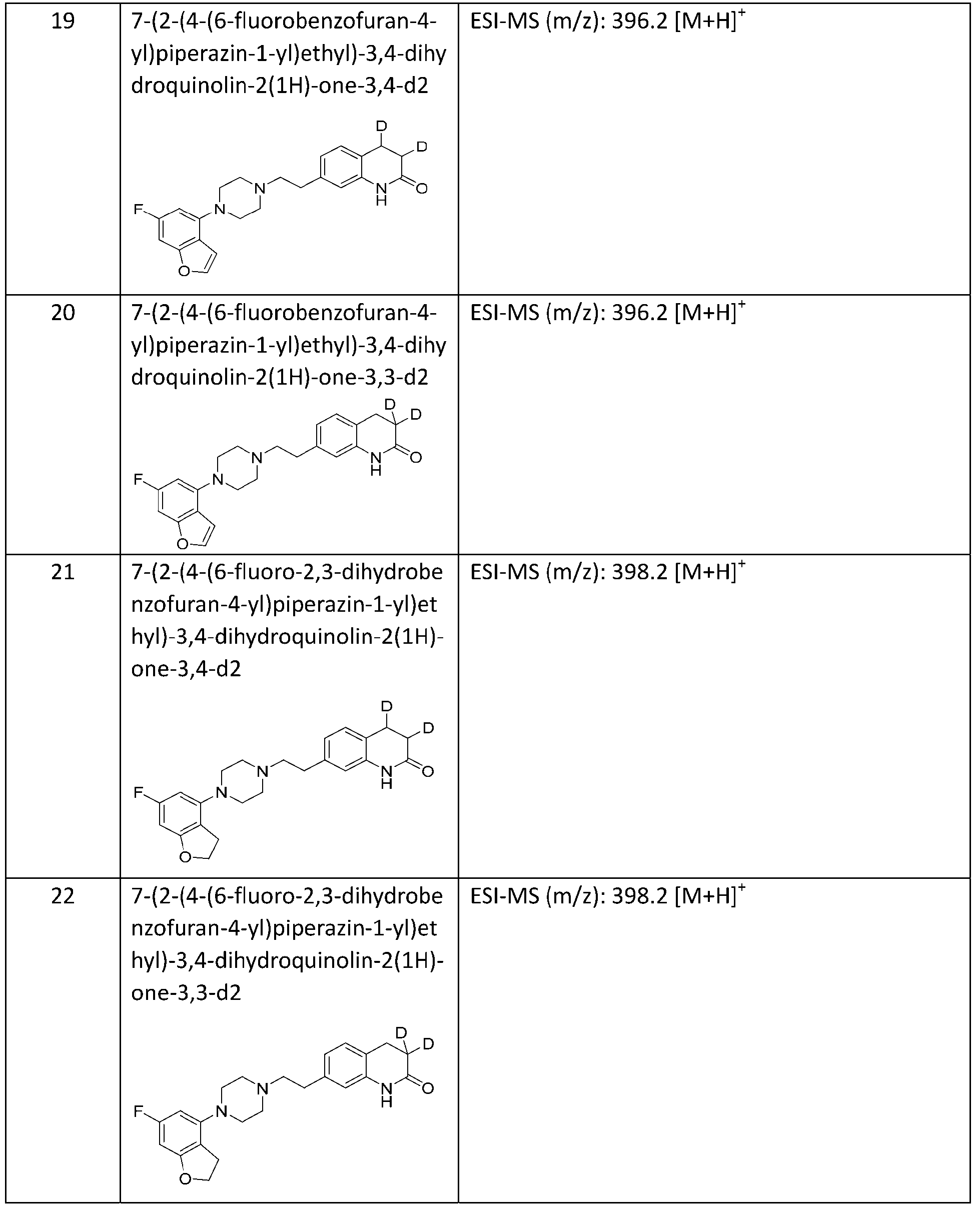

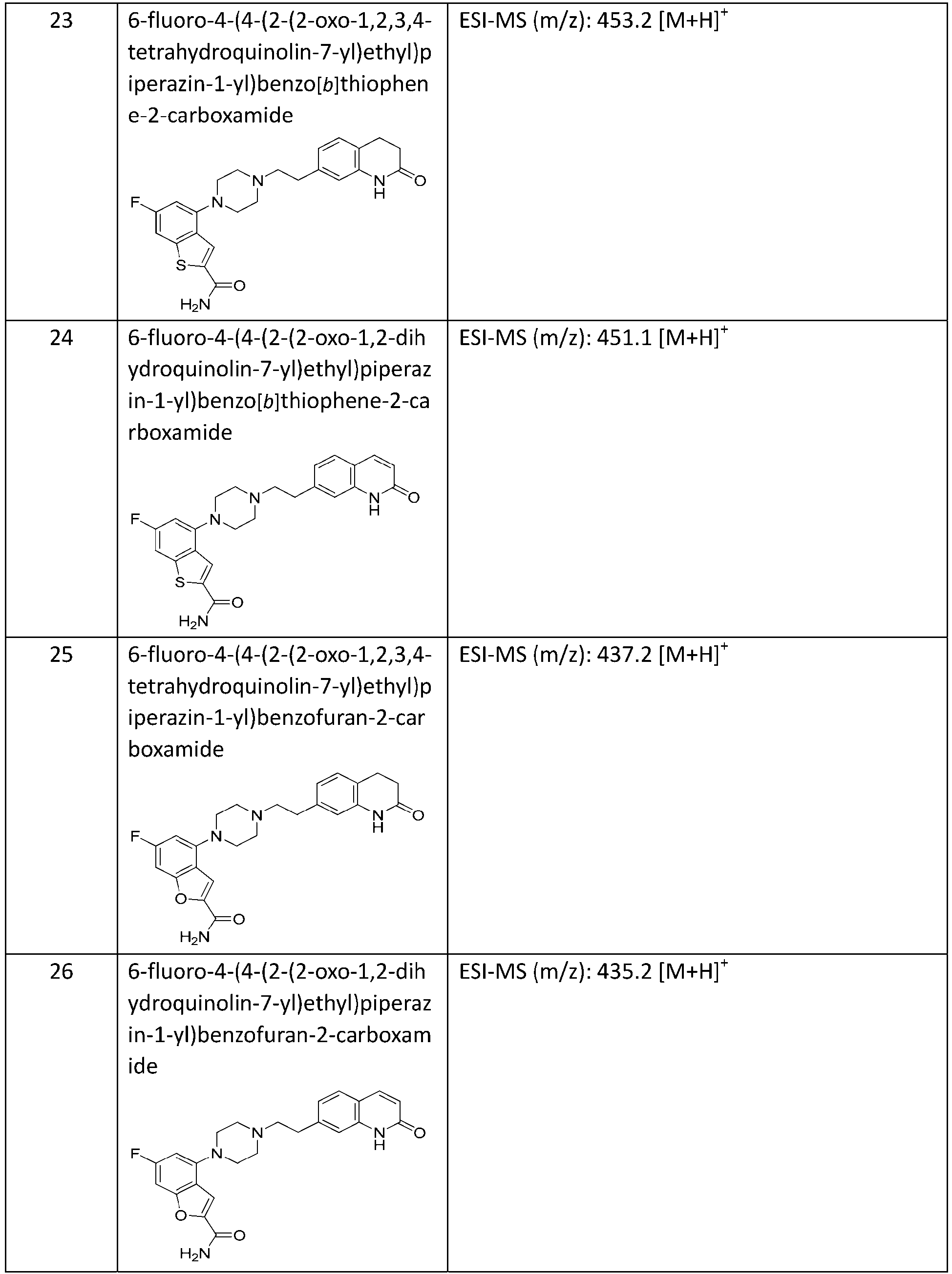

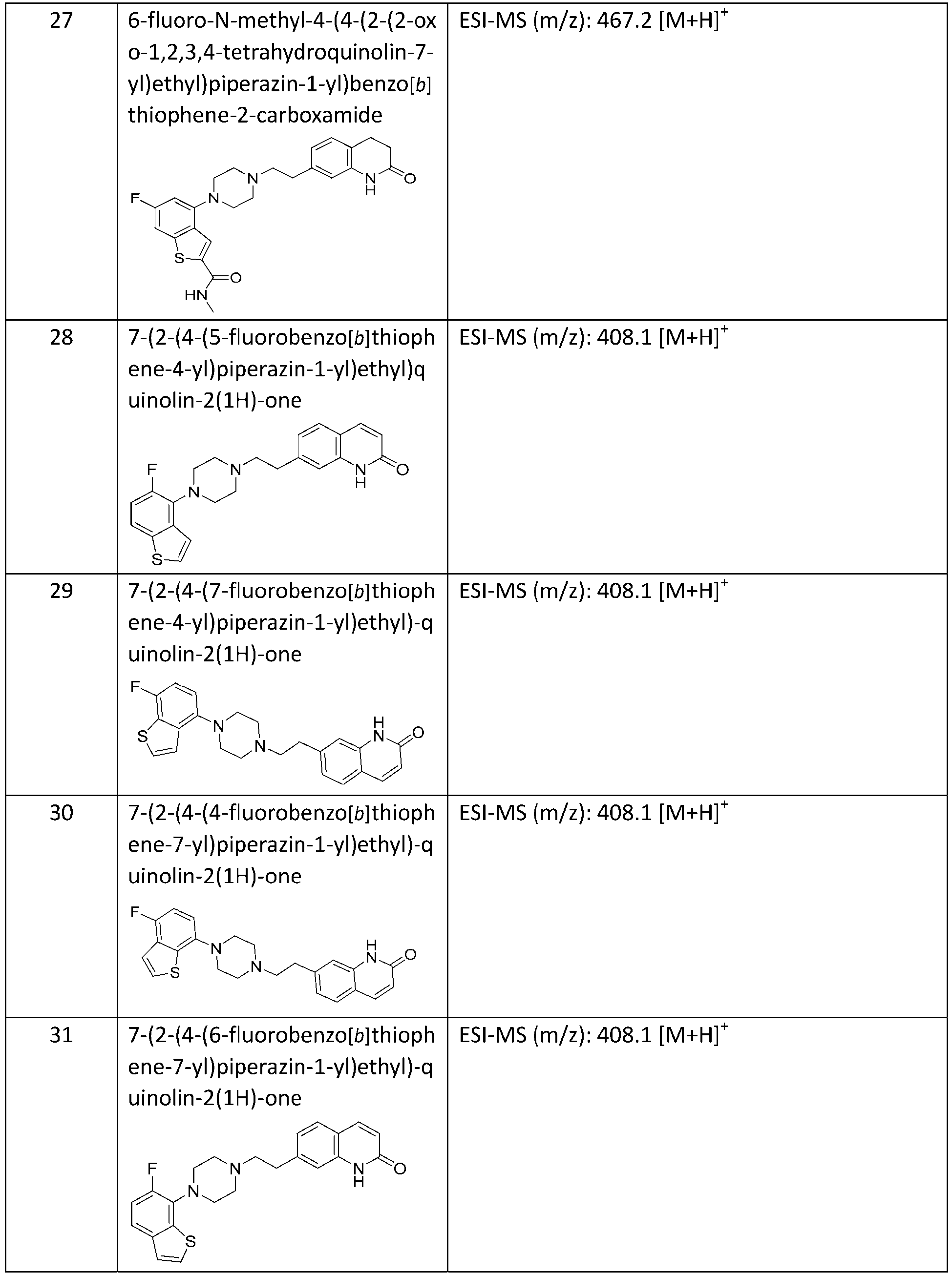

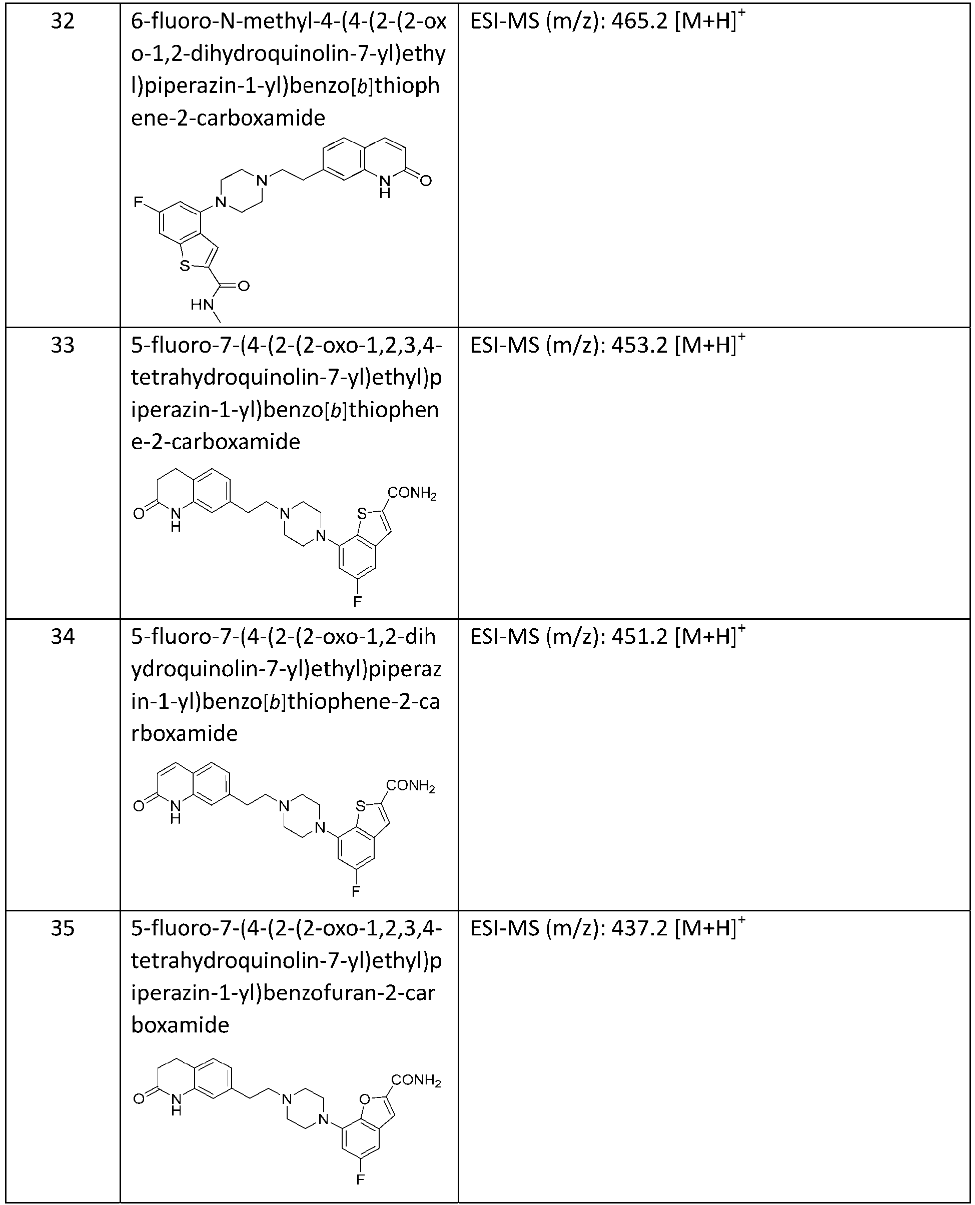

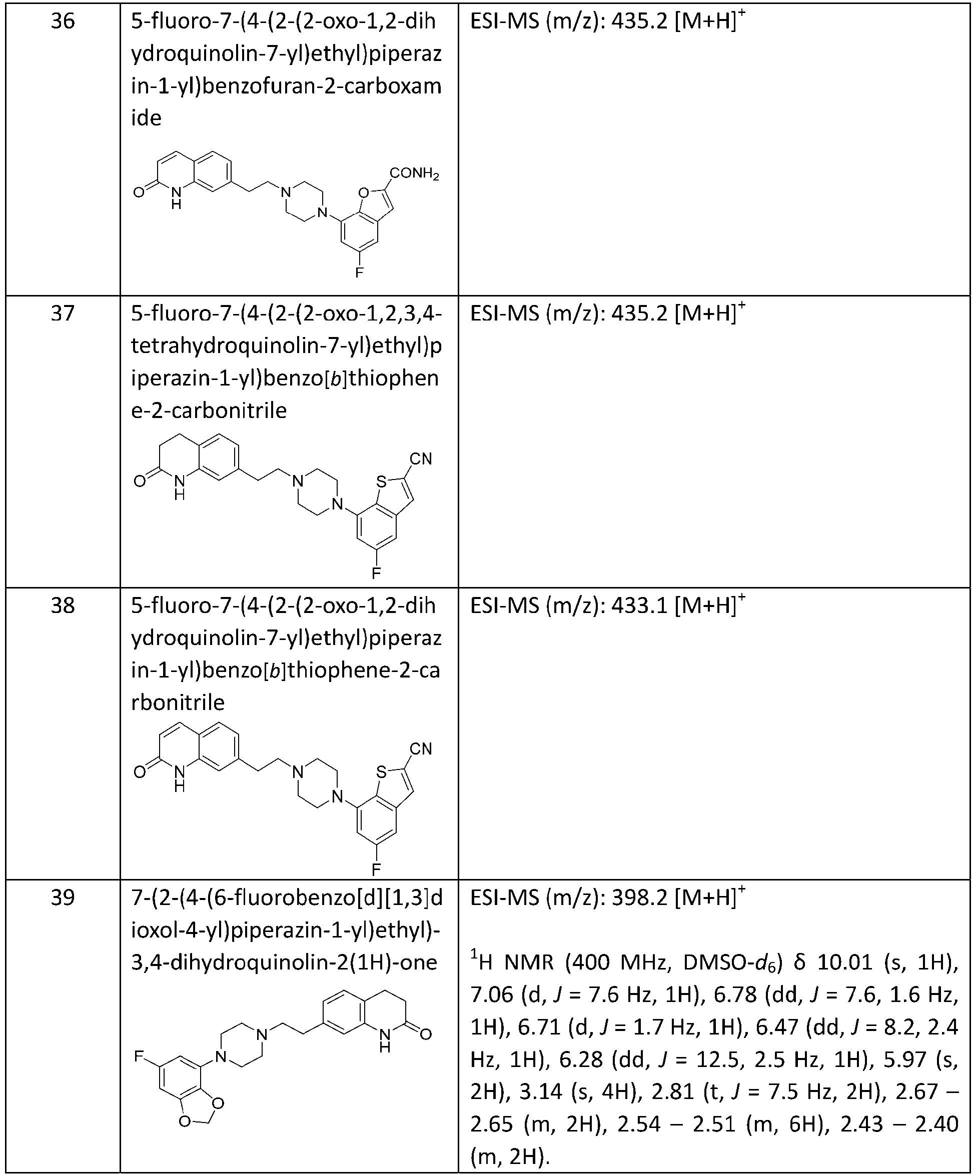

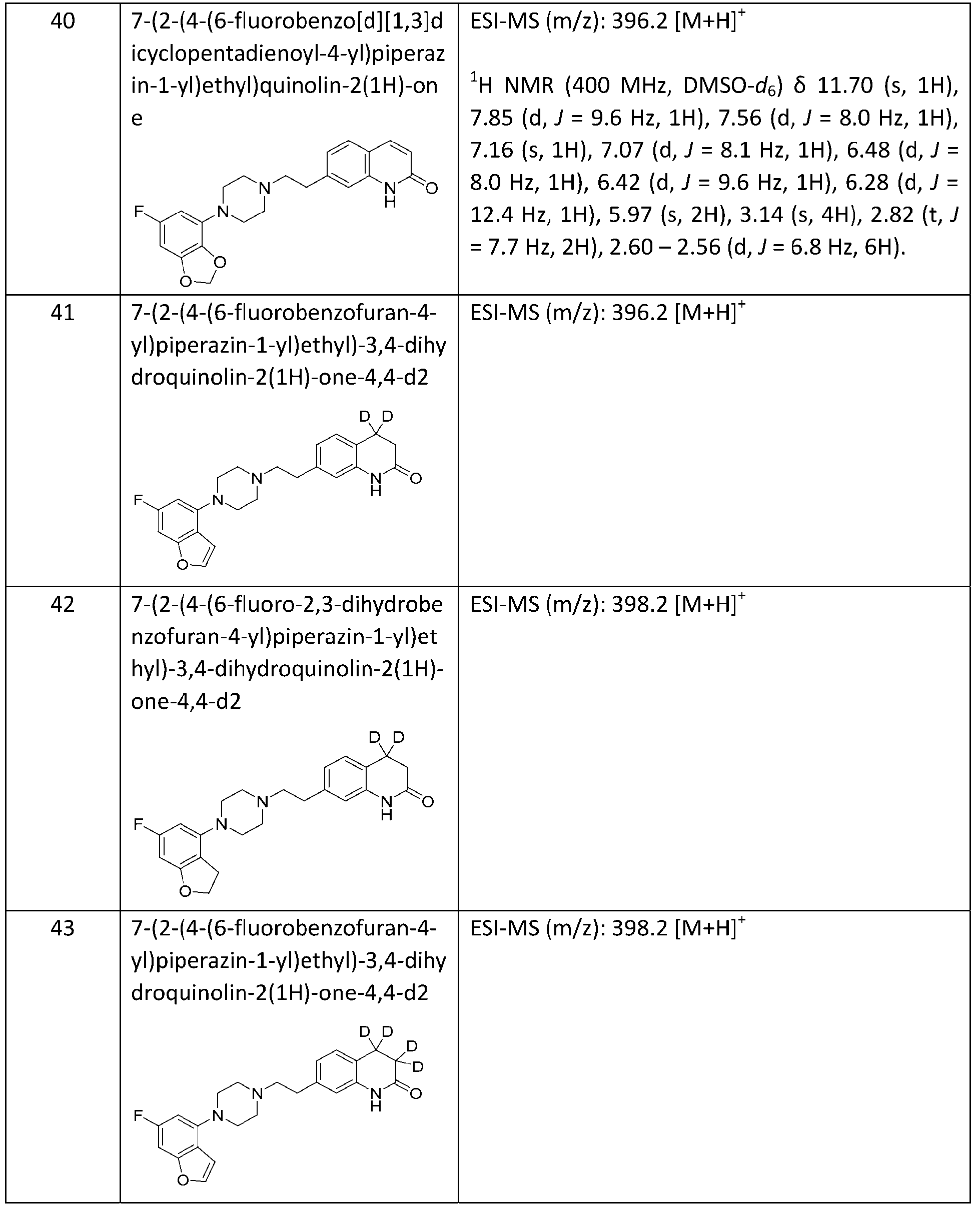

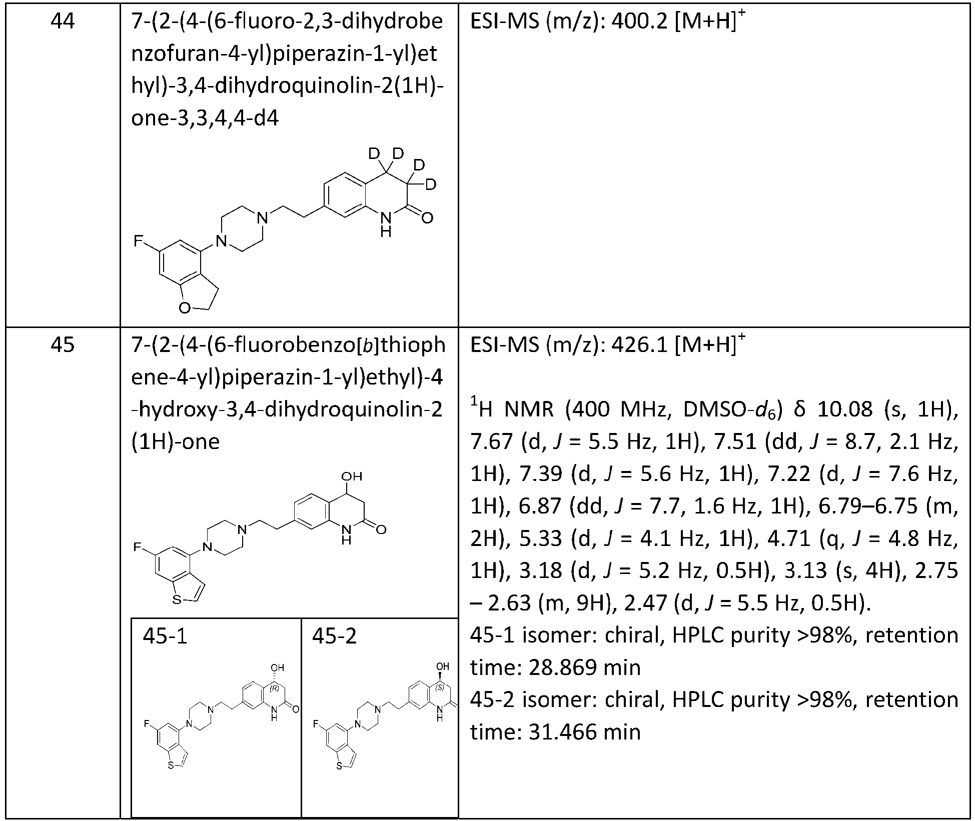

### Test Example 1. 5-HT_{1A} Receptor Agonist Activity Assay

The agonistic effect of the compounds on the 5-HT_{1A} receptor in CHO-K1 cells expressing the recombinant human 5-HT_{1A} receptor was tested using the LANCE^{™} cAMP 384 Kit (PerkinElmer, USA). The agonistic effect of the compounds on the 5-HT_{1A} receptor was evaluated by testing their inhibitory effect on cAMP production in CHO-K1 cells. 8-OH-DPAT was used as a positive control. The specific procedures were as follows:
a) The compounds (test compounds and positive control) were prepared to a stock concentration of 10 mM using DMSO, and Forskolin was prepared to a stock concentration of 10 mM. The initial test concentration of 8-OH-DPAT was 1 µM, diluted 3X. The initial test concentration of the test compounds was 0.1 µM, diluted 3X, and each concentration was repeated twice. 100 nL of the compounds and 10 nL of Forskolin were transferred to the test plate using an Echo, with a final DMSO concentration <1%.
b) The stable 5-HT_{1A} cell line was taken out from the incubator to observe the cell status. When the cell density reached 80%-90%, the culture medium was discarded and the cells were washed with 5 mL of DPBS. After discarding DPBS, 2 mL of trypsin was added, and the cells were incubated at 37 °C for 2-5 min. After adding 10 mL of complete culture medium, the cells were collected, which was centrifuged at 1000 rpm for 4 min, and the supernatant was discarded. The cell suspension was adjusted to a density of 1000 cells/well using Stimulation Buffer (HBSS + 5 mM HEPES + 0.5 mM IBMX + 0.1% BSA).
c) 10 µl of the prepared cell suspension was transferred to the assay plate preloaded with the compounds.
d) The cells were centrifuged at 600 rpm for 3 minutes and incubated at room temperature for 1 hour.
e) 5 µL of 4X Eu-cAMP tracer solution and 5 µL of 4X ULight^{™}-anti-cAMP solution were added, the cells were centrifuged at 600 rpm for 3 minutes, and incubated at room temperature for 1 hour.
f) Data were read using the EnVision multi-microplate reader, and EC₅₀ was calculated using Graphpad Prism software. The results are shown in Table 1.

**Table 1:**

| Test Compounds | 5-HT_{1A} agonistic activity (EC₅₀, nM) |
|---|---|
| Reference compound* | 1.0 |
| Compound of Example 1 | 0.5 |
| Compound of Example 2 | 1.0 |
| Compound of Example 3 | 1.3 |
| Compound of Example 5 | 1.5 |
| Compound of Example 12 | 0.8 |
| Compound of Example 14 | 1.0 |
| Compound of Example 15 | 0.5 |
| Compound of Example 16 | 0.5 |
| Compound of Example 41 | 0.7 |

| | |
|---|---|
| *Note: Compound (7-(2-(4-(6-fluorobenzo[*b*]thiophene-4-yl)piperazin-1-yl)ethyl)-3,4-dihydroquinolin-2(1H)-one) from Example 85 of patent application WO2015131856A1. | |

As shown in the table above, compounds 1-3, 5, 12, 14-16 of the present invention and the reference compound had comparable 5-HT_{1A} receptor agonist activity.

### Test Example 2. 5-HT Transporter Inhibitory Activity Assay

The inhibitory effect of the compounds on the 5-HT transporter in HEK-293 cells expressing human SERT was tested using the Neurotransmitter transporter uptake assay kit (Molecular Devices product). The test was performed according to the kit instructions, using citalopram as a positive control. The specific procedure was as follows:
a) HEK-hSERT cells were seeded at 20,000 cells/well in a 384-well plate and then incubated overnight at 37°C in an incubator.
b) The following day, the compounds (test compounds and positive control) were diluted using DMSO from 20 mM DMSO solution to the working concentration. Each test compound and citalopram were transferred to a 384-well plate using a detection buffer containing 0.1% BSA. The initial test concentration of citalopram was 1 µM, diluted 3X. The initial test concentration of the test compounds was 10 µM, diluted 4X, with each concentration repeated twice.
c) The cell culture 384-well plate was taken out from the incubator, the culture medium was aspirated from the wells, and 25 µL of the test compound solution was added per well. The cells were incubated at 37°C for 30 min. After incubation, 25 µL of a dye solution was added to each well and the cells were incubated at 37°C for 30 min.
d) the fluorescence values were read on a Flexstation 3 and the data were analyzed using Graphpad Prism. The results are shown in Table 2.

**Table 2:**

| Test Compounds | 5-HT uptake (IC₅₀, nM) |
|---|---|
| Compound of Example 1 | 7.8 |
| Compound of Example 2 | 3.6 |
| Compound of Example 5 | 13.0 |
| Compound of Example 6 | 12.0 |
| Compound of Example 7 | 9.0 |
| Compound of Example 15 | 26.0 |
| Compound of Example 22 | 12.4 |
| Compound of Example 39 | 10.6 |
| Compound of Example 43 | 7.4 |
| Reference Compound* | 15.0 |

| | |
|---|---|
| *Note: Compound of Example 85 in patent application WO2015131856A1. | |

As shown in the table above, the compounds in the examples of the present invention and the reference compound exhibited strong 5-HT transporter inhibitory activity, especially, the 5-HT transporter inhibitory activity of the compounds was even better, when Y in the general formula is an oxygen atom.

### Test Example 3. Mouse Pharmacokinetic Experiment

**Animal:** Male ICR mice. Animals were randomly assigned to the treatment group and fasted for 12 hours before administration.

**Drug to be tested:** The compounds were dissolved in solvent (0.5% CMC-Na). The intravenous (iv) dose was 0.3 mg/kg, with an administration volume of 5 ml/kg; the oral (po) dose was 1 mg/kg, with an administration volume of 10 mL/kg body weight.

**Sample Collection and Bioanalysis:** After administration, blood was collected from the orbital sinus of mice at 0.083, 0.25, 0.5, 1, 2, 4, 6, 8, and 24 hours (n=3 at each time point) and placed in EP tubes containing heparin sodium. Mice were euthanized immediately via vertebral dislocation after blood collection, and their brains were harvested at the corresponding time points. All samples were collected immediately, rapidly frozen, and stored at -70°C for later use.

Plasma samples were obtained by whole blood centrifugation. 10 µL of plasma was then mixed with 190 µL of internal standard solution (20 ng/mL metoprazor in acetonitrile containing 0.1% formic acid). After mixing, the mixture was centrifuged at 13000 rpm for 10 min. For each sample, 120 µL of the supernatant was collected, and 0.5-10 µL (depending on the sensitivity of the compound) was then taken for drug analysis using an appropriate liquid chromatography-tandem mass spectrometry (LC-MS/MS) method. Standards for each analyte were used for calibration and identification.

The brain tissue and extract were homogenized at a brain tissue:extract (50% acetonitrile-water) ratio = 1 (g): 8 (ml) using 5 3mm-magnetic beads in an OMNI Bead Ruptor 24 Elite homogenizer at 3.2 m/s for 2 min. The mixture was centrifuged at 4°C, 3000g for 3 min. 50 µL of supernatant was collected and added with 200 µL of internal standard solution (20 ng/mL dissolved in 80% acetonitrile-water). After mixing well, the mixture was centrifuged at 13000 rpm for 10 min. 120 µL of supernatant was collected for each sample. Then, 0.5-10 µL of the supernatant (depending on the sensitivity of the compound) was taken and analyzed using an appropriate LC-MS/MS method. Standards for each analyte were used for calibration and identification.

**Data Analysis:** Drug concentrations below the limit of quantitation (LLOQ) were recorded as zero. Pharmacokinetic data analysis was performed in PK Solver using non-compartmental, bolus intravenous injection, or extravascular administration analysis model. Data points below LLOQ were excluded from the analysis to improve the validity of t_{1/2} calculation. Plasma pharmacokinetic parameters are shown in Table 3 below, and Cₘₐₓ data in brain tissue are shown in Table 4:

**Table 3**

| Compounds | Cₘₐₓ(po)(ng/mL) | AUC₀₋ₜ₍ₚₒ₎ (ng*h/mL) | F(%) |
|---|---|---|---|
| Compound of Example 1 | 68.4 | 1170 | 100 |
| Compound of Example 2 | 72.8 | 794 | 77 |
| Reference Compound* | 27.5 | 468 | 50 |

**Table 4**

| Compounds | Cₘₐₓ(po)(ng/g) |
|---|---|
| Compound of Example 1 | 368 |
| Compound of Example 2 | 413 |
| Reference Compound* | 254 |

| | |
|---|---|
| *Note: The compound in Example 85 of patent application WO2015131856A1. | |

As shown in Tables 3 and 4 above, the compounds of Examples 1-2 of the present invention exhibited higher maximum oral plasma concentrations and bioavailability in mice compared to the reference compound. After oral administration at 1 mg/kg, the compounds of the present invention showed better brain penetration and higher brain concentrations in mice compared to the reference compound.

### Test Example 4. Rat Pharmacokinetic Experiment

The compound of Example 2 was used in a pharmacokinetic experiment in rats (non-fasted male SD rats). The compound of the present invention was dissolved in a solvent (0.5% CMC-Na). The intravenous (iv) dose was 1 mg/kg, with an administration volume of 5 ml/kg; the oral (po) dose was 3 mg/kg, with an administration volume of 10 mL/kg body weight. Approximately 0.3 mL of blood samples were collected from the jugular vein of rats at the following time points: 0.083, 0.25, 0.5, 1, 2, 4, 6, 8, and 24 h (n=3 at each time point). The collected blood was stored under ice-cooling conditions. Plasma was rapidly separated by centrifugation, and a precipitant was added. The concentration of the test compound was quantified using LC-MS. The experimental results are shown in Table 5 below:

**Table 5**

| Compound | Cₘₐₓ(po)(ng/mL) | AUC₀₋ₜ₍ₚₒ₎ (ng*h/mL) | F(%) |
|---|---|---|---|
| Compound of example 2 | 815 | 5324 | 100 |
| Reference Compound* | 280 | 1561 | 43 |

| | | | |
|---|---|---|---|
| *Note: The compound in Example 85 of patent application WO2015131856A1. | | | |

As shown in the table above, the compound in Example 2 of the present invention had a higher maximum oral plasma concentration and bioavailability in rats compared to the reference compound.

### Test Example 5. Forced Swim Test:

**Drugs to be tested:** The compounds of the present invention and the positive control compound (vortioxetine) were mixed with 5% DMSO, then mixed with 5% Solutol^{®} HS 15, and then 90% physiological saline was added to prepare a solution of appropriate concentration. The solution was prepared immediately before use.

**Animal:** Male C57 mice, approximately 22g. Animals were randomly divided into a blank control group and each test drug group, with 8 animals in each group. Mice in each group were intraperitoneally injected with either the solvent or the test drug.

**Experimental Procedure:** Forced swimming test was performed on the mice 0.5h after drug administration. The water level in the forced swimming device was 45cm, and the water temperature was 25°C. The mice were placed in the experimental room for 1h to acclimatize before the experiment. Mice were placed in the device at the start of the experiment for 6 minutes, and the entire process was recorded by a camera. Data analysis only considered the immobility time of the mice during the last 4 minutes. The compounds of the present invention exhibited significant antidepressant-like effects in the dose range of 0.01 to 0.1 mg/kg, with a low onset dose. Specifically, as shown in Figure 1, compound 2 (0.01 mg/kg) and the positive control vortioxetine (10 mg/kg) significantly reduced the immobility time of mice compared to the solvent control group, demonstrating antidepressant-like effects.

## Claims

1. A quinolinone compound represented by general formula (I), its stereoisomers, or a pharmaceutically acceptable salt thereof: 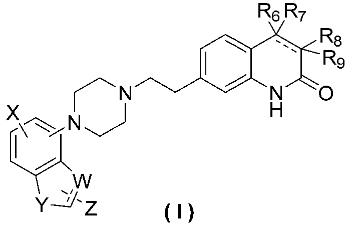 wherein
X on a benzene ring is a halogen;
Y is NH, O, or S;
W is CH, CH₂, or O;
------ represents a single bond or a double bond;
when W is CH₂ or O, the ------ connected to W represents a single bond; when W is CH, the
------ connected to W represents a double bond;
Z on a heterocycle is hydrogen, cyano, amino C1-C6 alkanoyl, or C1-C6 alkyl-substituted amino C1-C6 alkanoyl;
when the ------ connected to R₆, R₇, R₈, R₉ represents a single bond, R₆, R₇, R₈, and R₉ are each independently selected from the group consisting of hydrogen, deuterium, halogen, methyl, ethyl, hydroxyl, amino, and acetamido; or one of R₆ and R₇ as well as one of R₈ and R₉, along with the two carbon atoms connected with them, form a 3- to 6-membered carbon ring, and the other two substituents are defined as above;
when the ------ connected to R₆, R₇, R₈, R₉ represents a double bond, R₆ and R₈ are each independently selected from the group consisting of hydrogen, deuterium, halogen, methyl, ethyl, hydroxyl, amino, and acetamido, R₇ and R₉ are absent; alternatively, R₆ and R₈, along with the two carbon atoms connected with them, form a 3- to 6-membered carbon ring, and R₇ and R₉ are absent;
with condition that the following compounds are not included:
1) 7-(2-(4-(6-fluorobenzothiophene-4-yl)piperazin-1-yl)ethyl)-quinolin-2(1H)-one;
2) 7-(2-(4-(6-fluorobenzothiophene-4-yl)piperazin-1-yl)ethyl)-3,4-dihydroquinolin-2(1H)-one.

2. The quinolinone compound represented by general formula (I), its stereoisomers, or the pharmaceutically acceptable salt thereof according to claim 1, wherein,
X is F; and/or
Y is O or S; and/or
Z is hydrogen, cyano, amino C1-C4 alkanoyl, or C1-C4 alkyl-substituted amino C1-C4 alkanoyl (preferably N-methylcarbamoyl); preferably, Z is hydrogen, cyano, N-methylcarbamoyl, or carbamoyl; and/or
the ------ connected to R₆, R₇, R₈, and R₉ represents a single bond, R₆, R₇, R₈, and R₉ are each independently selected from the group consisting of hydrogen, deuterium, halogen, and hydroxyl; or R₆ and R₈ along with the two carbon atoms connected with them form a 3-membered carbon ring, R₇ and R₉ are hydrogen or deuterium; or the ------ connected to R₆, R₇, R₈, and R₉ represents a double bond, R₆ and R₈ are each independently selected from the group consisting of hydrogen, deuterium, halogen, and hydroxyl, and R₇ and R₉ are absent.

3. The quinolinone compound represented by general formula (I), its stereoisomers, or the pharmaceutically acceptable salt thereof according to claim 1, wherein,
------ represents a single or double bond;
X is a halogen;
Y is O or S;
W is CH, CH₂, or O;
Z is hydrogen, cyano, carbamoyl, or a C1-C6 alkyl-substituted carbamoyl group;
when the ------ connected to R₆, R₇, R₈, or R₉ represents a single bond, R₆, R₇, R₈, and R₉ are each independently selected from the group consisting of hydrogen, deuterium, halogen, methyl, ethyl, hydroxyl, amino, and acetamino; or one of R₆ and R₇ as well as one of R₈ or R₉, along with the two carbon atoms connected with them, form a 3- to 6-membered carbon ring, and the other two substituents are as defined above;
when the ------ connected to R₆, R₇, R₈, or R₉ represents a double bond, R₆ and R₈ are each independently selected from the group consisting of hydrogen, deuterium, halogen, methyl, ethyl, hydroxyl, amino, and acetamino, preferably hydrogen, deuterium, or halogen, and R₇ and R₉ are absent; or R₆ and R₈, along with the two carbon atoms connected with them, form a 4- to 6-membered carbon ring, and R₇ and R₉ are absent.

4. The quinolinone compound represented by general formula (I), its stereoisomers, or the pharmaceutically acceptable salt thereof according to claim 1, wherein,
------ represents a single or double bond;
X is F;
Y is O;
W is CH, CH₂, or O;
Z is hydrogen, cyano, carbamoyl, or a C1-C6 alkyl-substituted carbamoyl group;
when the ------ connected to R₆, R₇, R₈, R₉ represents a single bond, R₆, R₇, R₈, and R₉ are each independently selected from the group consisting of hydrogen, deuterium, halogen, methyl, ethyl, hydroxyl, amino, and acetamino; or one of R₆ and R₇ as well as one of R₈ and R₉, along with the two carbon atoms connected with them, form a 3- to 6-membered carbon ring, and the other two substituents are as defined above;
when the ------ connected to R₆, R₇, R₈, R₉ represents a double bond, R₆ and R₈ are each independently selected from the group consisting of hydrogen, deuterium, halogen, methyl, ethyl, hydroxyl, amino, and acetamino, preferably hydrogen, deuterium, or halogen, and R₇ and R₉ are absent; or R₆ and R₈, along with the two carbon atoms connected with them, form a 4- to 6-membered carbon ring, and R₇ and R₉ are absent.

5. The quinolinone compound represented by general formula (I), its stereoisomers, or the pharmaceutically acceptable salt thereof according to any one of claims 1 to 4, wherein, the quinolinone compound represented by general formula (I) is selected from the following structures: 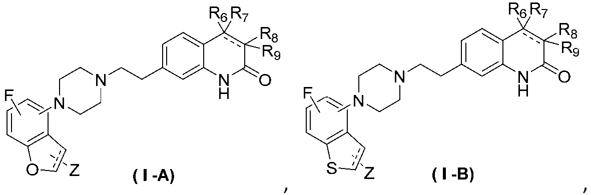 wherein, ------, Z, R₆, R₇, R₈, and R₉ are as defined in claims 1 to 4, respectively.

6. The quinolinone compound represented by general formula (I), its stereoisomers, or the pharmaceutically acceptable salt thereof according to any one of claims 1 to 4, wherein, the quinolinone compound represented by general formula (I) is selected from the following structures: 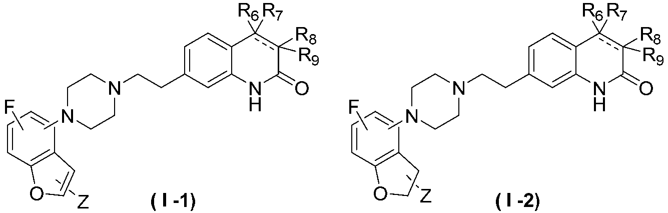 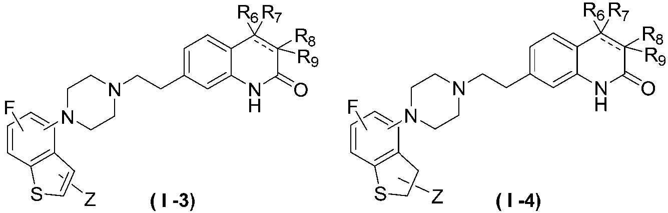 and 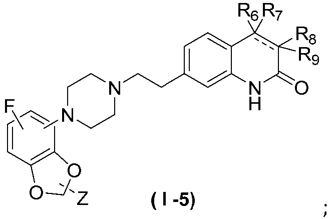
or, the quinolinone compound represented by general formula (I) is selected from the following structures: 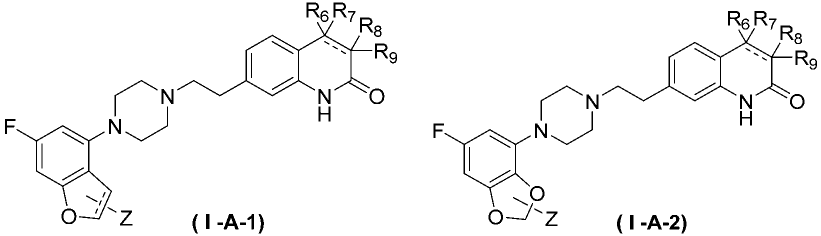 and 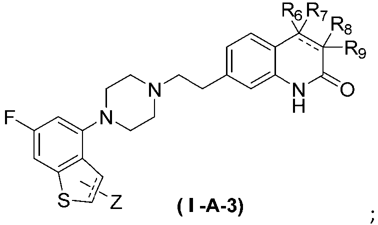
wherein, ------, Z, R₆, R₇, R₈, and R₉ are as defined in claims 1 to 4, respectively.

7. The quinolinone compound represented by general formula (I), its stereoisomers, or the pharmaceutically acceptable salt thereof according to claim 1, wherein, the quinolinone compound represented by general formula (I) is selected from the following compounds or pharmaceutically acceptable salts thereof:
(1) 7-(2-(4-(6-fluorobenzofuran-4-yl)piperazin-1-yl)ethyl)-3,4-dihydroquinolin-2(1H)-one; 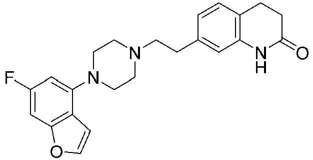
(2) 7-(2-(4-(6-fluoro-2,3-dihydrobenzofuran-4-yl)piperazin-1-yl)ethyl)-3,4-dihydroquinolin-2(1H)-one; 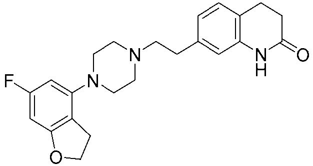
(3) 7-(2-(4-(5-fluorobenzofuran-7-yl)piperazin-1-yl)ethyl)quinolin-2(1H)-one; 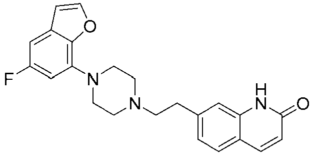
(4) 7-(2-(4-(5-fluoro-2,3-dihydrobenzofuran-7-yl)piperazin-1-yl)ethyl)quinolin-2(1H)-one; 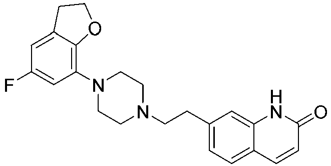
(5) 7-(2-(4-(5-fluoro-2,3-dihydrobenzofuran-7-yl)piperazin-1-yl)ethyl)-3,4-dihydroquinolin-2(1H)-one; 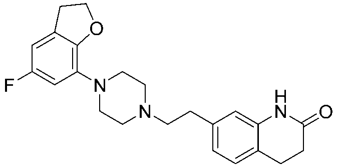
(6) 7-(2-(4-(5-fluorobenzofuran-7-yl)piperazin-1-yl)ethyl)-3,4-dihydroquinolin-2(1H)-one; 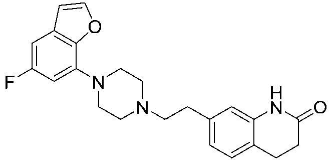
(7) 7-(2-(4-(6-fluoro-2,3-dihydrobenzofuran-4-yl)piperazin-1-yl)ethyl)quinolin-2(1H)-one; 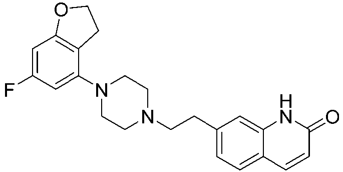
(8) 7-(2-(4-(6-fluorobenzofuran-4-yl)piperazin-1-yl)ethyl)quinolin-2(1H)-one; 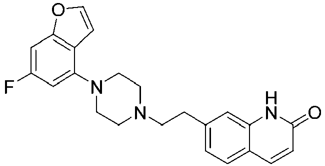
(9) 5-(2-(4-(6-fluorobenzo[b]thiophene-4-yl)piperazin-1-yl)ethyl)-1,1a,3,7b-tetrahydro-2H-cyclopropy l[*c*]quinolin-2-one; 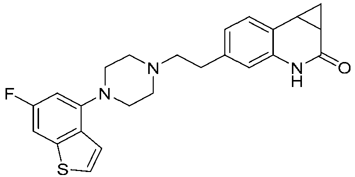
(10) 5-(2-(4-(5-fluorobenzo[b]thiophene-7-yl)piperazin-1-yl)ethyl)-1,1a,3,7b-tetrahydro-2H-cyclopropy l[*c*]quinolin-2-one; 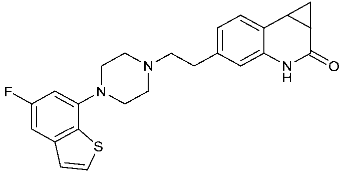
(11) 7-(2-(4-(5-fluorobenzo[b]thiophene-4-yl)piperazin-1-yl)ethyl)-3,4-dihydroquinolin-2(1H)-one; 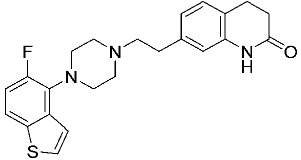
(12) 7-(2-(4-(7-fluorobenzo[b]thiophene-4-yl)piperazin-1-yl)ethyl)-3,4-dihydroquinolin-2(1H)-one; 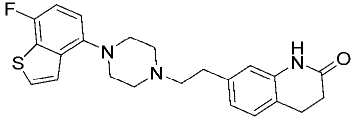
(13) 7-(2-(4-(4-fluorobenzo[*b*]thiophene-7-yl)piperazin-1-yl)ethyl)-3,4-dihydroquinolin-2(1H)-one; 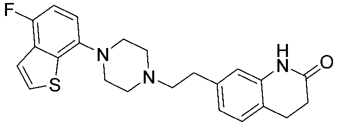
(14) 7-(2-(4-(6-fluorobenzo[*b*]thiophene-7-yl)piperazin-1-yl)ethyl)-3,4-dihydroquinolin-2(1H)-one; 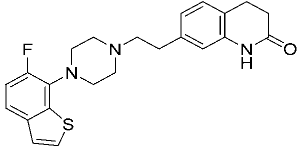
(15) 7-(2-(4-(5-fluorobenzo[*b*]thiophene-7-yl)piperazin-1-yl)ethyl)-3,4-dihydroquinolin-2(1H)-one; 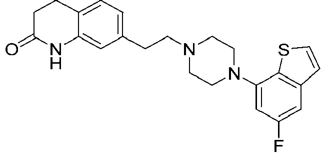
(16) 7-(2-(4-(5-fluorobenzo[*b*]thiophene-7-yl)piperazin-1-yl)ethyl)quinolin-2(1H)-one; 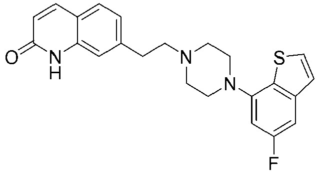
(17) 7-(2-(4-(6-fluorobenzofuran-4-yl)piperazin-1-yl)ethyl)-4-hydroxy-3,4-dihydroquinolin-2(1H)-one; 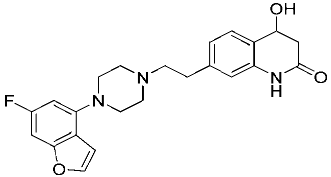
(18) 7-(2-(4-(6-fluoro-2,3-dihydrobenzofuran-4-yl)piperazin-1-yl)ethyl)-4-hydroxy-3,4-dihydroquinolin-2(1H )-one; 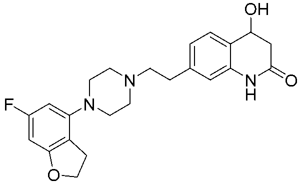
(19) 7-(2-(4-(6-fluorobenzofuran-4-yl)piperazin-1-yl)ethyl)-3,4-dihydroquinolin-2(1H)-one-3,4-d2; 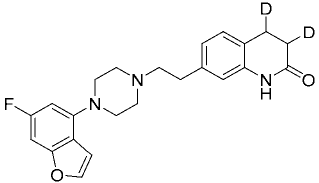
(20) 7-(2-(4-(6-fluorobenzofuran-4-yl)piperazin-1-yl)ethyl)-3,4-dihydroquinolin-2(1H)-one- 3,3-d2; 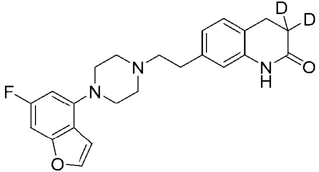
(21) **7-(2-(4-(6-fluoro-2,3-dihydrobenzofuran-4-yl)piperazin-1-yl)ethyl)-3,4-dihydroquinolin-2(1H)-one-**3,4-d2; 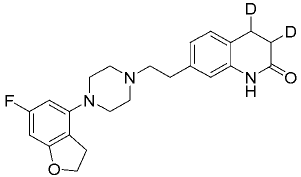
(22)7-(2-(4-(6-fluoro-2,3-dihydrobenzofuran-4-yl)piperazin-1-yl)ethyl)-3,4-dihydroquinolin-2(1H)-one-3,3-d2; 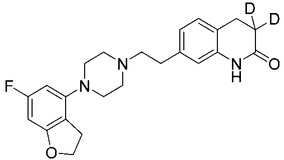
(23) 6-fluoro-4-(4-(2-(2-oxo-1,2, 3,4-tetrahydroquinolin-7-yl)ethyl)piperazin-1-yl)benzo[b]thiophene-2-carboxamide; 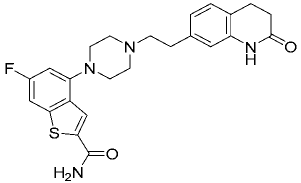
(24) 6-fluoro-4-(4-(2-(2-oxo-1,2-dihydroquinolin-7-yl)ethyl)piperazin-1-yl)benzo[b]thiophene-2-carbox amide; 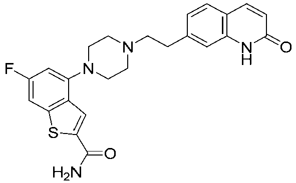
(25) 6-fluoro-4-(4-(2-(2-oxo-1,2,3,4-tetrahydroquinolin-7-yl)ethyl)piperazin-1-yl)benzofuran-2-carboxa mide; 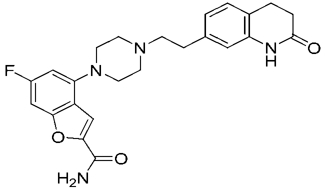
(26) 6-fluoro-4-(4-(2-(2-oxo-1,2-dihydroquinolin-7-yl)ethyl)piperazin-1-yl)benzofuran-2-carboxamide; 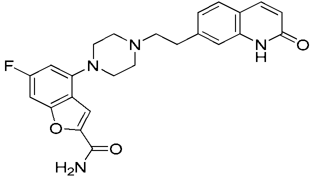
(27) 6-fluoro-N-methyl-4-(4-(2-(2-oxo-1,2,3,4-tetrahydroquinolin-7-yl)ethyl)piperazin-1-yl)benzo[b]thi ophene-2-carboxamide; 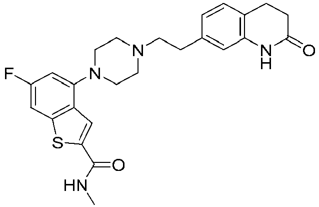
(28) 7-(2-(4-(5-fluorobenzo[b]thiophene-4-yl)piperazin-1-yl)ethyl)quinolin-2(1H)-one; 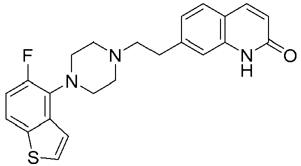
(29) 7-(2-(4-(7-fluorobenzo[b]thiophene-4-yl)piperazin-1-yl)ethyl)-quinolin-2(1H)-one; 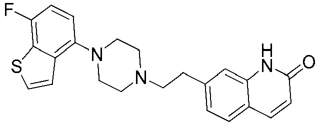
(30) 7-(2-(4-(4-fluorobenzo[b]thiophene-7-yl)piperazin-1-yl)ethyl)quinolin-2(1H)-one; 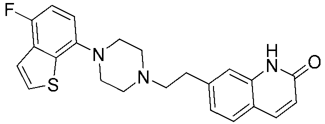
(31) 7-(2-(4-(6-fluorobenzo[*b*]thiophene-7-yl)piperazin-1-yl)ethyl)quinolin-2(1H)-one; 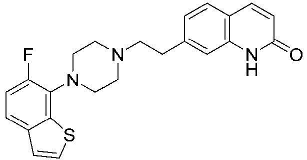
(32) 6-fluoro-N-methyl-4-(4-(2-(2-oxo-1,2-dihydroquinolin-7-yl)ethyl)piperazin-1-yl)benzo[b]thiophene -2-carboxamide; 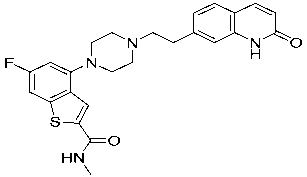
(33) 5-fluoro-7-(4-(2-(2-oxo-1,2,3,4-tetrahydroquinolin-7-yl)ethyl)piperazin-1-yl)benzo[b]thiophene-2-carboxamide; 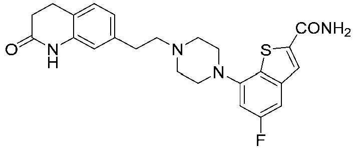
(34) 5-fluoro-7-(4-(2-(2-oxo-1,2-dihydroquinolin-7-yl)ethyl)piperazin-1-yl)benzo[*b*]thiophene-2-carbox amide; 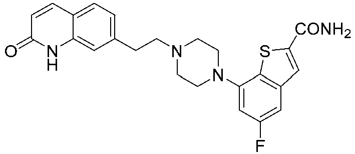
(35) 5-fluoro-7-(4-(2-(2-oxo-1,2,3,4-tetrahydroquinolin-7-yl)ethyl)piperazin-1-yl)benzofuran-2-carboxa mide; 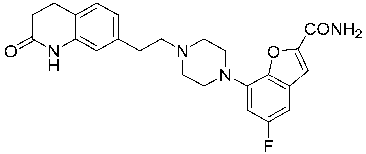
(36) 5-fluoro-7-(4-(2-(2-oxo-1,2-dihydroquinolin-7-yl)ethyl)piperazin-1-yl)benzofuran-2-carboxamide; 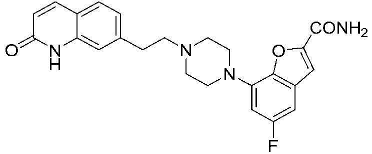
(37) 5-fluoro-7-(4-(2-(2-oxo-1,2,3,4-tetrahydroquinolin-7-yl)ethyl)piperazin-1-yl)benzo[b]thiophene-2-carbonitrile; 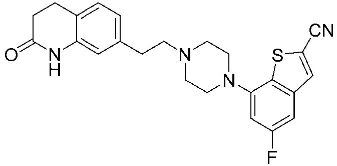
(38) 5-fluoro-7-(4-(2-(2-oxo-1,2-dihydroquinolin-7-yl)ethyl)piperazin-1-yl)benzo[b]thiophene-2-carbon itrile; 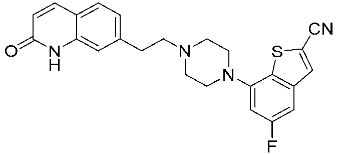
(39) 7-(2-(4-(6-fluorobenzo[d][1,3]dioxol-4-yl)piperazin-1-yl)ethyl)-3,4-dihydroquinolin-2(1H)-one; 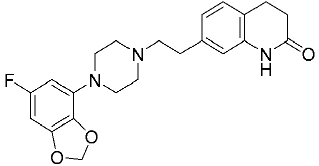
(40) 7-(2-(4-(6-fluorobenzo[d][1,3]dioxol-4-yl)piperazin-1-yl)ethyl)quinolin-2(1H)-one; 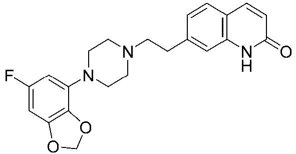
(41) 7-(2-(4-(6-fluorobenzofuran-4-yl)piperazin-1-yl)ethyl)-3,4-dihydroquinolin-2(1H)-one-4,4-d2; 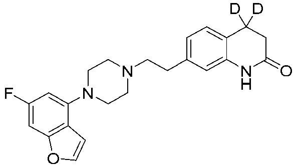
(42) 7-(2-(4-(6-fluoro-2,3-dihydrobenzofuran-4-yl)piperazin-1-yl)ethyl)-3,4-dihydroquinolin-2(1H)-one-4,4-d2; 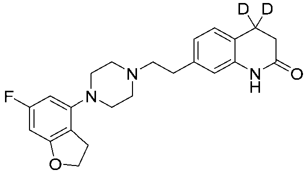
(43) 7-(2-(4-(6-fluorobenzofuran-4-yl)piperazin-1-yl)ethyl)-3,4-dihydroquinolin-2(1H)-one-3,3,4,4-d4; 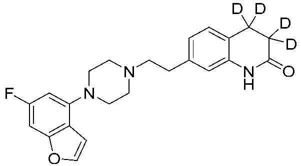
(44) 7-(2-(4-(6-fluoro-2,3-dihydrobenzofuran-4-yl)piperazin-1-yl)ethyl)-3,4-dihydroquinolin-2(1H)-one-3,3,4,4-d4; 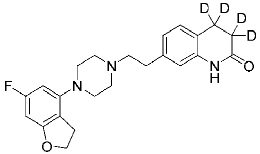
(45) 7-(2-(4-(6-fluorobenzo[b]thiophene-4-yl)piperazin-1-yl)ethyl)-4-hydroxy-3,4-dihydroquinolin-2(1H )-one; 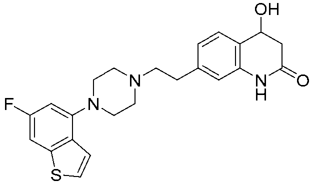
(45-1)R-7-(2-(4-(6-fluorobenzo[*b*]thiophene-4-yl)piperazin-1-yl)ethyl)-4-hydroxy-3,4-dihydroquino lin-2(1H)-one; 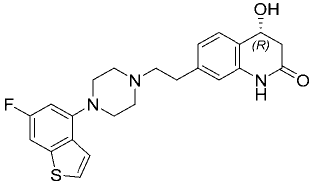 and
(45-2)S-7-(2-(4-(6-fluorobenzo[*b*]thiophene-4-yl)piperazin-1-yl)ethyl)-4-hydroxy-3,4-dihydroquino lin-2(1H)-one;

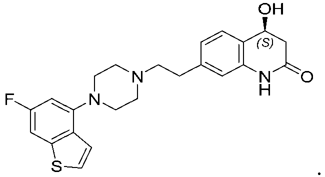

8. A method for preparing the quinolinone compound represented by general formula (I) of any one of claims 1 to 7, which is carried out by one of the following methods 1 and 2:
Method 1:
a compound represented by formula (II) or a salt thereof is reacted with a compound represented by formula (III) or a salt thereof via an N-alkylation reaction, as shown in reaction scheme 1: 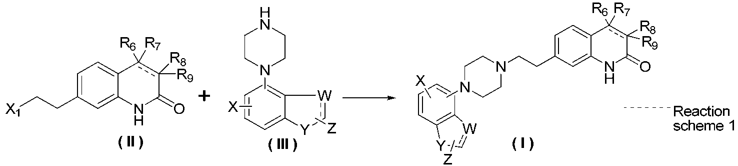
wherein, ------, X, Y, R₆, R₇, R₈, and R₉ are as defined in corresponding claims;
X₁ represents a leaving group, such as halogen, C1-C6 alkylsulfonyloxy, benzenesulfonyloxy, or naphthalenesulfonyloxy, wherein the C1-C6 alkylsulfonyloxy, benzenesulfonyloxy, and naphthalenesulfonyloxy may be optionally further substituted with one or more groups selected from the group consisting of halogen, hydroxyl, amino, nitro, C1-C6 alkyl, C1-C6 alkoxy, and C1-C6 alkanoyl;
preferably, the reaction is carried out with or without a solvent, or in a solvent with or without a base;
the reaction temperature is between room temperature to 200°C, and the reaction time is 1 to 30 hours;
Method 2:
a compound represented by formula (IV) or a salt thereof is reacted with a compound represented by formula (V) or a salt thereof via a coupling reaction, as shown in reaction scheme 2: 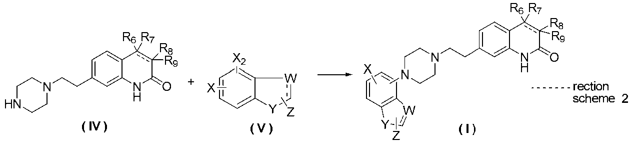
wherein, ------, X, Y, W, Z, R₆, R₇, R₈, and R₉ are as defined in corresponding claims;
X₂ represents a halogen or trifluoromethanesulfonyloxy group,
preferably, the reaction is carried out in the presence of a palladium catalyst and a base, with or without a solvent;
preferably, the reaction temperature is between room temperature to 200°C, and the reaction time is 1 to 30 hours.

9. A pharmaceutical composition, comprising a therapeutically effective amount of the quinolinone compound represented by general formula (I), its stereoisomer or the pharmaceutically acceptable salt thereof of any one of claims 1 to 7, and optionally a pharmaceutically acceptable carrier.

10. Use of the quinolinone compound represented by general formula (I), its stereoisomers, or pharmaceutically acceptable salts thereof of any one of claims 1 to 7, or the pharmaceutical composition of claim 9, in the preparation of a medicament for the prevention and/or treatment of central nervous system diseases;
preferably, the central nervous system diseases are selected from: schizophrenia; affective disorders; mental disorders; mood disorders; type I bipolar disorder; type II bipolar disorder; depression; dysphoric disorder; cyclothymic disorder; panic disorder; obsessive-compulsive disorder; impulsivity disorder; post-traumatic stress disorder; anxiety disorder; hysteria; anorexia nervosa; sleep disorders; adjustment disorders; cognitive impairment; autism; nervous headache; mania; Parkinson's disease; Huntington's disease; various dementias; memory impairment; ADHD; attention deficit/hyperactivity disorder and tic disorders;
more preferably, the central nervous system diseases are selected from: refractory, difficult-to-treat, or chronic schizophrenia; endogenous depression; major depressive disorder; refractory depression; panic attacks; social phobia; acute stress disorder; and Alzheimer's disease.
